# EUROPEAN PATENT APPLICATION

(11) **EP 4 401 186 A1**
(43) Date of publication of application: **17.07.2024**
(21) Application number: 22881012.3
(22) Date of filing: 11.10.2022
(51) Int. Cl.: H01M 10/0567, C07C 311/48, H01M 10/052, H01M 10/054, H01M 10/0568, H01M 10/0569

(54) **NON-AQUEOUS ELECTROLYTIC SOLUTION, NON-AQUEOUS ELECTROLYTE BATTERY, AND COMPOUND**

(30) Priority: 12.10.2021 JP 2021167757
(71) Applicant: Central Glass Company, Limited, Ube-shi, Yamaguchi 755-0001 (JP)
(72) Inventor: NAKAHARA Keita, Tokyo 101-0054 (JP); SHIMIZU Genki, Tokyo 101-0054 (JP); MORINAKA Takayoshi, Tokyo 101-0054 (JP)
(74) Representative: Manitz Finsterwald Patent- und Rechtsanwaltspartnerschaft mbB
(86) International application number: PCT/JP2022/037891
(87) International publication number: WO 2023/063317

(57) **Abstract**

The present disclosure provides a nonaqueous electrolyte solution containing: (I) at least one selected from the group consisting of a compound represented by Formula (1) described in the specification, a compound represented by Formula (2) described in the specification, and a compound represented by Formula (3) described in the specification, and a nonaqueous electrolyte solution battery including at least a positive electrode, a negative electrode, a separator, and the above nonaqueous electrolyte solution.

## Description

### TECHNICAL FIELD

The present disclosure relates to a nonaqueous electrolyte solution, a nonaqueous electrolyte solution battery, and a compound.

### BACKGROUND ART

As measures for improving cycle characteristics, high-temperature storage characteristics, and durability of a nonaqueous electrolyte solution battery, optimization of various constituting elements of the battery including active materials of a positive electrode and a negative electrode has been hitherto studied. Techniques relating to a nonaqueous electrolyte solution have also been studied, and use of a variety of additives to prevent deterioration of a nonaqueous electrolyte solution at surfaces of active positive and negative electrodes due to decomposition of the electrolyte solution has been proposed.

Patent Literature 1 discloses a nonaqueous electrolyte solution in which an electrolyte is dissolved in a nonaqueous solvent as the nonaqueous electrolyte solution that can provide a nonaqueous electrolyte solution battery with excellent initial resistance characteristics, and the nonaqueous electrolyte solution further contains a specific compound having a sulfonylamide structure.

### CITATION LIST

### PATENT LITERATURE

Patent Literature 1: WO2021/015264

### SUMMARY OF INVENTION

### TECHNICAL PROBLEM

However, the present inventors investigated and found that the addition of the specific compound having a sulfonylamide structure as described in Patent Literature 1 improved the initial resistance characteristics, but there is still room for investigation regarding an effect of suppressing an increase in resistance during a charge and discharge cycle in a high-temperature environment.

The present disclosure is made in view of the above circumstances, and an object thereof is to provide a nonaqueous electrolyte solution and a nonaqueous electrolyte solution battery that can reduce an initial resistance value and suppress an increase in resistance during a high-temperature cycle. Another object is to provide a compound that can be suitably used in the above nonaqueous electrolyte solution.

### SOLUTION TO PROBLEM

The present inventors conducted intensive research in view of such a problem and found that a nonaqueous electrolyte solution battery that can reduce an initial resistance value and suppress an increase in resistance during a high-temperature cycle can be obtained by using a nonaqueous electrolyte solution containing: (I) at least one selected from the group consisting of a compound represented by Formula (1) described later, a compound represented by Formula (2) described later, and a compound represented by Formula (3) described later (hereinafter, also described as "Component (I)"), thereby solving the above problem.

That is, the present inventors found that the above problem can be solved by the following configurations.

[1] A nonaqueous electrolyte solution, including:
   (I) at least one selected from the group consisting of a compound represented by the following Formula (1), a compound represented by the following Formula (2), and a compound represented by the following Formula (3). 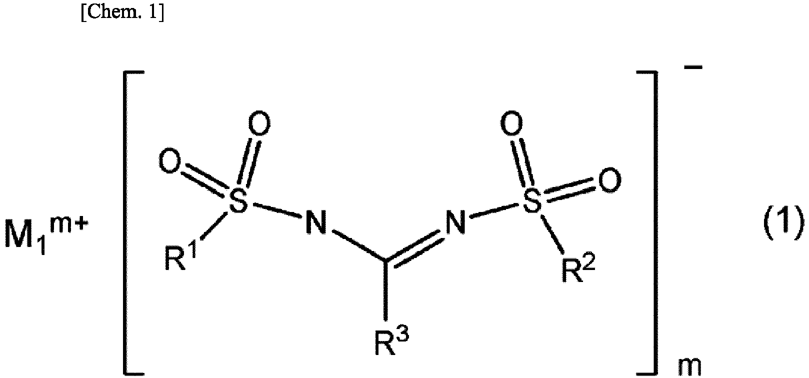 [In the Formula (1), M₁^{m+} represents a proton, a metal cation, or an onium cation, and m represents a valence of the cation.
      R¹ and R² each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).
      R³ represents a halogen atom, a perfluoroalkyl group having 1 to 4 carbon atoms, a perfluoroalkenyl group having 2 to 4 carbon atoms, a perfluoroalkynyl group having 2 to 4 carbon atoms, a cyano group, an amino group, an aminocarbonyl group, a perfluoroalkylcarbonyl group having 2 to 4 carbon atoms, a hydrocarbon group having 1 to 10 carbon atoms (the hydrocarbon group may have a hetero atom or a halogen atom, and may have a branched chain or a cyclic structure if the number of carbon atoms is 3 or more), -C(=O)-R' (R' represents a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms (the alkoxy group, alkenyloxy group, and alkynyloxy group described above may further have a hetero atom or a halogen atom, and may have a branched chain or cyclic structure if the number of carbon atoms is 3 or more), an aryloxy group having 6 to 10 carbon atoms, or -O-(Mₐ^{p+})_{q}, Mₐ^{p+} represents a proton, a metal cation, or an onium cation, and p represents a valence of the cation, q represents a number to satisfy p × q = 1), -R^{a}-S(=O)₂(R^{b}), or -R^{c}-P(=O)(R^{d})(R^{c}).
      R^{a} and R^{c} each independently represent a perfluoroalkylene group having 1 to 4 carbon atoms or a hydrocarbon group having 1 to 10 carbon atoms (the hydrocarbon group may have a hetero atom or a halogen atom, and may have a branched chain or cyclic structure if the number of carbon atoms is 3 or more).
      R^{b} represents a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₂^{r+})ₛ, M₂^{r+} represents a proton, a metal cation, or an onium cation, and r represents a valence of the cation. s represents a number to satisfy r × s = 1.
      R^{d} and R^{e} each independently represent a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₃^{t+})ᵤ, M₃^{t+} represents a proton, a metal cation, or an onium cation, and t represents a valence of the cation. u represents a number to satisfy t × u = 1.] 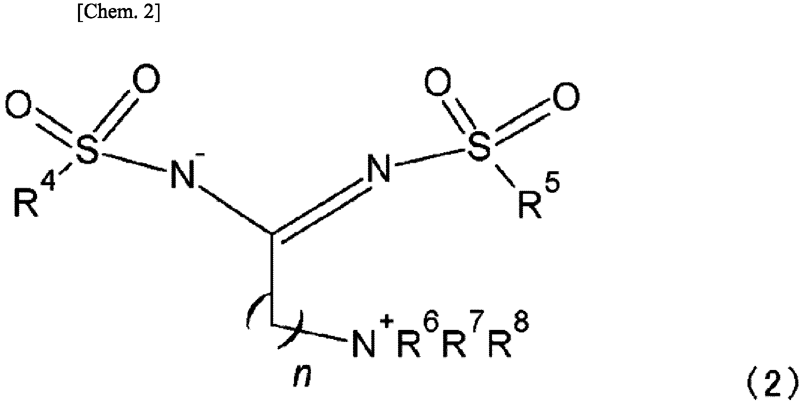 [in Formula (2), R⁴ and R⁵ each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).
         R⁶ to R⁸ each independently represent a hydrogen atom or a hydrocarbon group having 1 to 10 carbon atoms (the hydrocarbon group may have a hetero atom or a halogen atom, and may have a branched chain or cyclic structure if the number of carbon atoms is 3 or more).
         n represents an integer of 1 to 4.] 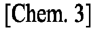 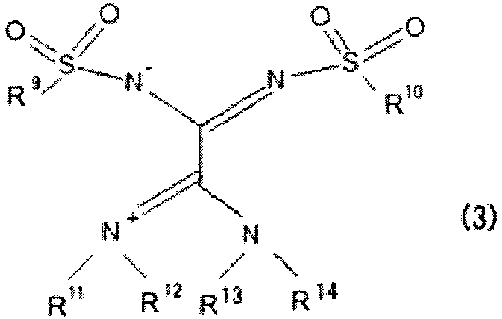 [in Formula (3), R⁹ and R¹⁰ each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).
            R¹¹ to R¹⁴ each independently represent a hydrogen atom or a hydrocarbon group having 1 to 10 carbon atoms (the hydrocarbon group may have a hetero atom or a halogen atom, and may have a branched chain or cyclic structure if the number of carbon atoms is 3 or more). R¹² and R¹³ may bond to each other to form a ring.]
[2] The nonaqueous electrolyte solution according to [1], in which at least one of R¹ and R² in the Formula (1) represents a fluorine atom.
[3] The nonaqueous electrolyte solution according to [1] or [2], in which at least one of R⁴ and R⁵ in the Formula (2) represents a fluorine atom.
[4] The nonaqueous electrolyte solution according to any one of [1] to [3], in which at least one of R⁹ and R¹⁰ in the Formula (3) represents a fluorine atom.
[5] The nonaqueous electrolyte solution according to any one of [1] to [4], further including: (II) a solute.
[6] The nonaqueous electrolyte solution according to [1] to [5], further including: (III) a nonaqueous organic solvent.
[7] The nonaqueous electrolyte solution according to [5], in which the (II) solute is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlC_{b}, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.
[8] The nonaqueous electrolyte solution according to [6], in which the (III) nonaqueous organic solvent includes at least one selected from the group consisting of a cyclic ester, a chain ester, a cyclic ether, a chain ether, a sulfone compound, a sulfoxide compound, and an ionic liquid.
[9] The nonaqueous electrolyte solution according to [8], in which the nonaqueous organic solvent includes a cyclic ester, and the cyclic ester includes cyclic carbonate.
[10] The nonaqueous electrolyte solution according to [9], in which the cyclic carbonate includes at least one selected from the group consisting of ethylene carbonate, propylene carbonate, and fluoroethylene carbonate.
[11] The nonaqueous electrolyte solution according to [8], in which the nonaqueous organic solvent includes a chain ester, and the chain ester includes chain carbonate.
[12] The nonaqueous electrolyte solution according to [11], in which the chain carbonate includes at least one selected from the group consisting of ethyl methyl carbonate, dimethyl carbonate, diethyl carbonate, and methyl propyl carbonate.
[13] The nonaqueous electrolyte solution according to any one of [1] to [12], in which a content of the (I) is 0.01% by mass to 5.0% by mass with respect to the total amount of the nonaqueous electrolyte solution.
[14] The nonaqueous electrolyte solution according to any one of [1] to [13], further including: at least one selected from vinylene carbonate, bis(oxalato)borate, difluorooxalatoborate, difluorobis(oxalato)phosphate, tetrafluorooxalatophosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene.
[15] A nonaqueous electrolyte solution battery, at least including: a positive electrode; a negative electrode; a separator; and the nonaqueous electrolyte solution according to any one of [1] to [14].
[16] A compound represented by the following Formula (1), a compound represented by the following Formula (2), or a compound represented by the following Formula (3). 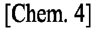 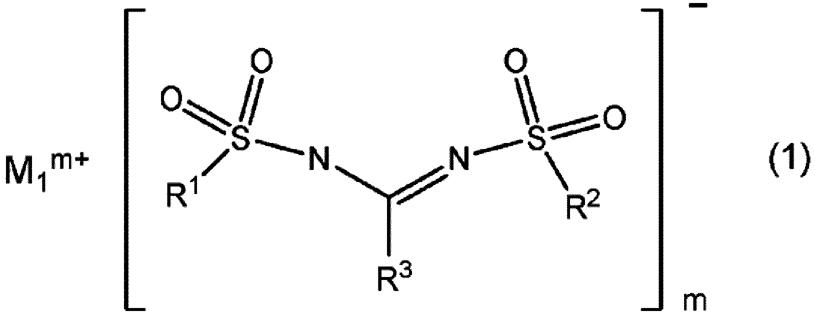 [in Formula (1), M₁^{m+} represents a proton, a metal cation, or an onium cation, and m represents a valence of the cation.
   R¹ and R² each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).
   R³ represents a halogen atom, a perfluoroalkyl group having 1 to 4 carbon atoms, a perfluoroalkenyl group having 2 to 4 carbon atoms, a perfluoroalkynyl group having 2 to 4 carbon atoms, a cyano group, an amino group, an aminocarbonyl group, a perfluoroalkylcarbonyl group having 2 to 4 carbon atoms, a hydrocarbon group having 1 to 10 carbon atoms (the hydrocarbon group may have a hetero atom or a halogen atom, and may have a branched chain or a cyclic structure if the number of carbon atoms is 3 or more), -C(=O)-R' (R' represents a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms (the alkoxy group, alkenyloxy group, and alkynyloxy group described above may further have a hetero atom or a halogen atom, and may have a branched chain or cyclic structure if the number of carbon atoms is 3 or more), an aryloxy group having 6 to 10 carbon atoms, or -O-(Mₑ^{p+})_{q}, Mₐ^{p+} represents a proton, a metal cation, or an onium cation, and p represents a valence of the cation, q represents a number to satisfy p × q = 1), -R^{a}-S(=O)₂(R^{b}), or -R^{c}-P(=O)(R^{d})(R^{e}).
   R^{a} and R^{c} each independently represent a perfluoroalkylene group having 1 to 4 carbon atoms or a hydrocarbon group having 1 to 10 carbon atoms (the hydrocarbon group may have a hetero atom or a halogen atom, and may have a branched chain or cyclic structure if the number of carbon atoms is 3 or more).
   R^{b} represents a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₂^{r+})ₛ, M₂^{r+} represents a proton, a metal cation, or an onium cation, and r represents a valence of the cation, s represents a number to satisfy r × s = 1.
   R^{d} and R^{e} each independently represent a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₃^{t+})ᵤ, M₃^{t+} represents a proton, a metal cation, or an onium cation, and t represents a valence of the cation. u represents a number to satisfy t × u = 1.] 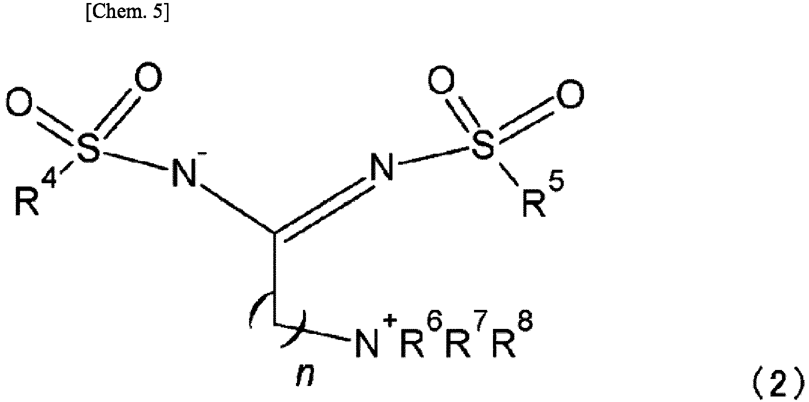
      [0034] [in Formula (2), R⁴ and R⁵ each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).
      R⁶ to R⁸ each independently represent a hydrogen atom or a hydrocarbon group having 1 to 10 carbon atoms (the hydrocarbon group may have a hetero atom or a halogen atom, and may have a branched chain or cyclic structure if the number of carbon atoms is 3 or more).
      n represents an integer of 1 to 4.] 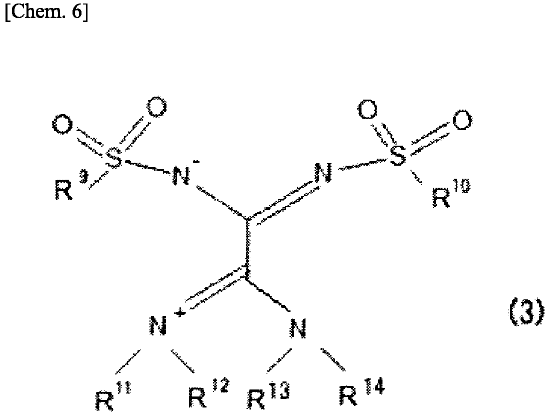
      [in Formula (3), R⁹ and R¹⁰ each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more),
      R¹¹ to R¹⁴ each independently represent a hydrogen atom or a hydrocarbon group having 1 to 10 carbon atoms (the hydrocarbon group may have a hetero atom or a halogen atom, and may have a branched chain or cyclic structure if the number of carbon atoms is 3 or more). R¹² and R¹³ may bond to each other to form a ring.]
[17] The compound according to [16], in which at least one of R¹ and R² in the Formula (1) represents a fluorine atom.
[18] The compound according to [16], in which at least one of R⁴ and R⁵ in the Formula (2) represents a fluorine atom.
[19] The compound according to [16], in which at least one of R⁹ and R¹⁰ in the Formula (3) represents a fluorine atom.

### ADVANTAGEOUS EFFECTS OF INVENTION

The present disclosure allows for providing a nonaqueous electrolyte solution and a nonaqueous electrolyte solution battery that can reduce an initial resistance value and suppress an increase during a high-temperature cycle. The present disclosure also allows for providing a compound that can be suitably used in the above nonaqueous electrolyte solution.

### DESCRIPTION OF EMBODIMENTS

Configurations and combinations thereof in the following embodiments are merely examples, and configuration additions, replacements, and other changes are possible without departing from the scope of the present disclosure. In addition, the present disclosure is not limited by the embodiments, but only by the scope of claims.

Ranges expressed with "to" in the present specification mean ranges including numerical values indicated before and after "to" as a lower limit value and an upper limit value.

An initial resistance value in the present specification represents a resistance value of a nonaqueous electrolyte solution battery immediately after an initial charge and discharge operation for battery stabilization. Specifically, the initial resistance value refers to a resistance value obtained by initial impedance measurement after three cycles of the charge and discharge operation for battery stabilization.

### [1. Nonaqueous Electrolyte Solution]

A nonaqueous electrolyte solution according to the present disclosure is a nonaqueous electrolyte solution containing (I) at least one selected from the group consisting of a compound represented by the above Formula (1), a compound represented by the above Formula (2), and a compound represented by the above Formula (3).

### <Component (I)>

The nonaqueous electrolyte solution according to the present disclosure contains at least one selected from the group consisting of the compound represented by Formula (1), the compound represented by Formula (2), and the compound represented by Formula (3), which is Component (I).

When the nonaqueous electrolyte solution containing the above Component (I) is used in a nonaqueous electrolyte solution battery (for example, a lithium ion secondary battery or a sodium ion secondary battery), Component (I) decomposes on at least one of a positive electrode and a negative electrode, and forms a film having good cation conductivity on a surface of at least one of the positive electrode and the negative electrode. It is considered that this film prevents direct contact between a nonaqueous organic solvent or a solute and an electrode active material, and reduces cation dissociation energy of the solute. The present inventors estimate that the film results in an effect of reducing an initial resistance of a nonaqueous electrolyte solution battery and allows for suppressing an increase in resistance during a high-temperature cycle.

The compound represented by Formula (1) is described below.

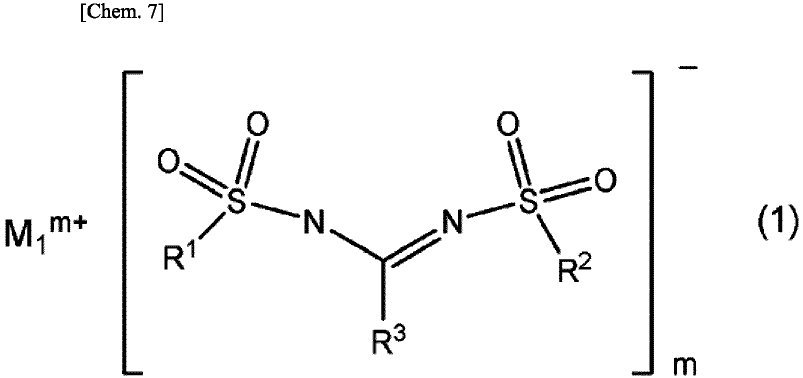

In Formula (1), M₁^{m+} represents a proton, a metal cation, or an onium cation, and m represents a valence of the cation.

R¹ and R² each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).

R³ represents a halogen atom, a perfluoroalkyl group having 1 to 4 carbon atoms, a perfluoroalkenyl group having 2 to 4 carbon atoms, a perfluoroalkynyl group having 2 to 4 carbon atoms, a cyano group, an amino group, an aminocarbonyl group, a perfluoroalkylcarbonyl group having 2 to 4 carbon atoms, a hydrocarbon group having 1 to 10 carbon atoms (the hydrocarbon group may have a hetero atom or a halogen atom, and may have a branched chain or a cyclic structure if the number of carbon atoms is 3 or more), -C(=O)-R' (R' represents a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms (the alkoxy group, alkenyloxy group, and alkynyloxy group described above may further have a hetero atom or a halogen atom, and may have a branched chain or cyclic structure if the number of carbon atoms is 3 or more), an aryloxy group having 6 to 10 carbon atoms, or -O-(Mₐ^{p+})_{q}, Mₐ^{p+} represents a proton, a metal cation, or an onium cation, and p represents a valence of the cation. q represents a number to satisfy p × q = 1), -R^{a}-S(=O)₂(R^{b}), or -R^{c}-P(=O)(R^{d})(R^{e}).

R^{a} and R^{c} each independently represent a perfluoroalkylene group having 1 to 4 carbon atoms or a hydrocarbon group having 1 to 10 carbon atoms (the hydrocarbon group may have a hetero atom or a halogen atom, and may have a branched chain or cyclic structure if the number of carbon atoms is 3 or more).

R^{b} represents a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₂^{r+})ₛ, M₂^{r+} represents a proton, a metal cation, or an onium cation, and r represents a valence of the cation. s represents a number to satisfy r × s = 1.

R^{d} and R^{e} each independently represent a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₃^{t+})ᵤ, M₃^{t+} represents a proton, a metal cation, or an onium cation, and t represents a valence of the cation, u represents a number to satisfy t × u = 1.

In the present specification, a hydrocarbon group refers to a group having a CH structure in which a carbon atom and a hydrogen atom are bonded.

In Formula (1), M₁^{m+} represents a proton, a metal cation, or an onium cation, and m represents a valence of the cation.

When M₁^{m+} represents a metal cation, examples of the metal cation include an alkali metal cation such as a lithium ion, a sodium ion, and a potassium ion, and an alkaline earth metal cation such as a magnesium ion and a calcium ion.

When M₁^{m+} represents an onium cation, examples of the onium cation include a trialkylammonium ion, a tetraalkylammonium ion, a tetraalkylphosphonium ion, an imidazolium ion, and an ammonium ion having a spiro skeleton.

m represents the valence of the cation. When M₁^{m+} represents a cation with a valence of 1, m is 1, and when M₁^{m+} represents a cation with a valence of 2, m is 2.

M₁^{m+} may represent a metal cation, may be a lithium ion or a sodium ion, or may be a lithium ion.

In Formula (1), R¹ and R² each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).

When R¹ and R² represent a halogen atom, examples of the halogen atom include a fluorine atom, a chlorine atom, and an iodine atom.

When R¹ and R² represent a perfluoroalkyl group having 1 to 4 carbon atoms, examples of the perfluoroalkyl group include a trifluoromethyl group, a pentafluoroethyl group, a heptafluoropropyl group, and a heptafluoroisopropyl group.

When R¹ and R² represent a hydrocarbon group having 1 to 10 carbon atoms, examples of the hydrocarbon group include but not limited to an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 1 to 10 carbon atoms in which these groups are combined.

When R¹ and R² represent an alkyl group having 1 to 10 carbon atoms, examples of the alkyl group include a methyl group, an ethyl group, an n-propyl group, an isopropyl group, an n-butyl group, a sec-butyl group, an isobutyl group, a tert-butyl group, an n-pentyl group, an n-hexyl group, an n-octyl group, and an n-decyl group.

When R¹ and R² represent an alkenyl group having 2 to 10 carbon atoms, examples of the alkenyl group include a vinyl group, an allyl group, a 1-propenyl group, an isopropenyl group, a 2-butenyl group, and a 1,3-butadienyl group.

When R¹ and R² represent an alkynyl group having 2 to 10 carbon atoms, examples of the alkynyl group include an ethynyl group, a 1-propynyl group, a 2-propynyl group, and a 1,1-dimethyl-2-propynyl group.

When R¹ and R² represent a cycloalkyl group having 3 to 10 carbon atoms, examples of the cycloalkyl group include a cyclopentyl group and a cyclohexyl group.

When R¹ and R² represent a cycloalkenyl group having 3 to 10 carbon atoms, examples of the cycloalkenyl group include a cyclopentenyl group and a cyclohexenyl group.

When R¹ and R² represent an aryl group having 6 to 10 carbon atoms, examples of the aryl group include a phenyl group and a naphthyl group.

The above hydrocarbon group may have a hetero atom. Examples of the hetero atom include an oxygen atom, a sulfur atom, and a nitrogen atom.

Any hydrogen atom of the above hydrocarbon group may be substituted with a halogen atom. Examples of the halogen atom include a fluorine atom, a chlorine atom, and an iodine atom, and the halogen atom may be a fluorine atom.

At least one of R¹ and R² may represent a halogen atom, at least one thereof may represent a fluorine atom, or both may represent a fluorine atom.

In Formula (1), R³ represents a halogen atom, a perfluoroalkyl group having 1 to 4 carbon atoms, a perfluoroalkenyl group having 2 to 4 carbon atoms, a perfluoroalkynyl group having 2 to 4 carbon atoms, a cyano group, an amino group, an aminocarbonyl group, a perfluoroalkylcarbonyl group having 2 to 4 carbon atoms, a hydrocarbon group having 1 to 10 carbon atoms (the hydrocarbon group may have a hetero atom or a halogen atom, and may have a branched chain or a cyclic structure if the number of carbon atoms is 3 or more), -C(=O)-R' (R' represents a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms (the alkoxy group, alkenyloxy group, and alkynyloxy group described above may further have a hetero atom or a halogen atom, and may have a branched chain or cyclic structure if the number of carbon atoms is 3 or more), an aryloxy group having 6 to 10 carbon atoms, or -O-(Mₐ^{p+})_{q}, Mₐ^{p+} represents a proton, a metal cation, or an onium cation, and p represents a valence of the cation. q represents a number to satisfy p × q = 1), -R^{a}-S(=O)₂(R^{b}), or -R^{c}-P(=O)(R^{d})(R^{e}).

R^{a} and R^{c} each independently represent a perfluoroalkylene group having 1 to 4 carbon atoms or a hydrocarbon group having 1 to 10 carbon atoms (the hydrocarbon group may have a hetero atom or a halogen atom, and may have a branched chain or cyclic structure if the number of carbon atoms is 3 or more).

R^{b} represents a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₂^{r+})ₛ, M₂^{r+} represents a proton, a metal cation, or an onium cation, and r represents a valence of the cation. s represents a number to satisfy r × s = 1.

R^{d} and R^{e} each independently represent a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₃^{t+})ᵤ, M₃^{t+} represents a proton, a metal cation, or an onium cation, and t represents a valence of the cation. u represents a number to satisfy t × u = 1.

When R³ represents a halogen atom, examples of the halogen atom include a fluorine atom, a chlorine atom, and an iodine atom.

When R³ represents a perfluoroalkyl group having 1 to 4 carbon atoms, examples of the perfluoroalkyl group include a trifluoromethyl group, a pentafluoroethyl group, a heptafluoropropyl group, and a heptafluoroisopropyl group.

When R³ represents a perfluoroalkenyl group having 2 to 4 carbon atoms, examples of the perfluoroalkenyl group include a -CF=CF₂ group, a -CF₂CF=CF₂ group, and a -CF=CFCF₃ group.

When R³ represents a perfluoroalkynyl group having 2 to 4 carbon atoms, examples of the perfluoroalkynyl group include a fluoroethynyl group and a 3,3,3-trifluoropropynyl group.

When R³ represents a perfluoroalkylcarbonyl group having 2 to 4 carbon atoms, examples of the perfluoroalkylcarbonyl group include a -C(=O)CF₃ group and a -C(=O)CF₂CF₃ group.

When R³ represents a hydrocarbon group having 1 to 10 carbon atoms, examples of the hydrocarbon group include but not limited to an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 1 to 10 carbon atoms in which these groups are combined.

Specific examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms include examples of the above R¹ and R² for an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms.

The above hydrocarbon group may have a hetero atom. Examples of the hetero atom include an oxygen atom, a sulfur atom, and a nitrogen atom, and specific examples of the hydrocarbon group having a hetero atom include the following groups of 1) to 4). 1) A group in which any hydrogen atom of the above hydrocarbon group having 1 to 10 carbon atoms is substituted with a hetero atom-containing group such as a cyano group, a thiol group, or an amino group
2) A group in which any carbon atom in the above hydrocarbon group having 1 to 10 carbon atoms is a carbonyl carbon (C=O)
3) A heterocyclic group having 3 to 10 carbon atoms
4) A group containing a hetero atom between any carbon atom-carbon atom bond in the above hydrocarbon group having 1 to 10 carbon atoms

In the above 2), examples of the group in which any carbon atom in the hydrocarbon group is a carbonyl carbon (C=O) include an alkylcarbonyl group having 2 to 10 carbon atoms, an alkenylcarbonyl group having 3 to 10 carbon atoms, an alkynylcarbonyl group having 3 to 10 carbon atoms, a cycloalkylcarbonyl group having 4 to 10 carbon atoms, a cycloalkenylcarbonyl group having 4 to 10 carbon atoms, and an arylcarbonyl group having 7 to 10 carbon atoms.

In the above 3), the heterocyclic group having 3 to 10 carbon atoms may be a saturated heterocyclic group or an unsaturated heterocyclic group.

The saturated heterocyclic group is preferably a 5-membered or 6-membered saturated heterocyclic group, and examples thereof include a group obtained by removing one hydrogen atom from tetrahydrofuran, pyrrolidine, tetrahydropyran, or piperidine.

The unsaturated heterocyclic group is preferably a 5-membered or 6-membered unsaturated heterocyclic group, and examples thereof include a group obtained by removing one hydrogen atom from furan, oxazole, isoxazole, pyrrole, thiophene, pyridine, pyrazine, or pyrimidine.

The carbon atoms forming the heterocycle may be carbonyl carbons, and for example, the heterocyclic group may be a cyclic ester group.

The above hydrocarbon group may have a halogen atom. In other words, any hydrogen atom of the above hydrocarbon group may be substituted with a halogen atom. Examples of the halogen atom include a fluorine atom, a chlorine atom, and an iodine atom, and the halogen atom may be a fluorine atom.

The hydrocarbon group may have a substituent. Examples of the substituent include but not limited to an alkyl group and a halogen atom.

The number of carbon atoms in the hydrocarbon group including a substituent is 1 to 10.

When R' represents a hydrocarbon group having 1 to 10 carbon atoms, examples of the hydrocarbon group include but not limited to an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 1 to 10 carbon atoms in which these groups are combined.

Specific examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms include examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms in the above R¹ and R².

The above hydrocarbon group may have a hetero atom. Examples of the hetero atom include an oxygen atom, a sulfur atom, and a nitrogen atom, and specific examples of the hydrocarbon group having a hetero atom include the above groups of 1) to 4).

The above hydrocarbon group may have a halogen atom. In other words, any hydrogen atom of the above hydrocarbon group may be substituted with a halogen atom. Examples of the halogen atom include a fluorine atom, a chlorine atom, and an iodine atom, and the halogen atom may be a fluorine atom.

The hydrocarbon group may have a substituent. Examples of the substituent include but not limited to an alkyl group and a halogen atom.

The number of carbon atoms in the hydrocarbon group including a substituent is 1 to 10.

When R' represents an alkoxy group having 1 to 10 carbon atoms, examples of the alkyl group having 1 to 10 carbon atoms in the alkoxy group having 1 to 10 carbon atoms are the same as those of the alkyl group having 1 to 10 carbon atoms in the hydrocarbon group having 1 to 10 carbon atoms in the above R¹ and R².

When R' represents an alkenyloxy group having 2 to 10 carbon atoms, examples of the alkenyl group having 2 to 10 carbon atoms in the alkenyloxy group having 2 to 10 carbon atoms are the same as those of the alkenyl group having 2 to 10 carbon atoms in the hydrocarbon group having 1 to 10 carbon atoms in the above R¹ and R².

When R' represents an alkynyloxy group having 2 to 10 carbon atoms, examples of the alkynyl group having 2 to 10 carbon atoms in the alkynyloxy group having 2 to 10 carbon atoms are the same as those of the alkynyl group having 2 to 10 carbon atoms in the hydrocarbon group having 1 to 10 carbon atoms in the above R¹ and R².

When R' represents an aryloxy group having 6 to 10 carbon atoms, examples of the aryl group having 6 to 10 carbon atoms in the aryloxy group having 6 to 10 carbon atoms are the same as those of the aryl group having 6 to 10 carbon atoms in the hydrocarbon group having 1 to 10 carbon atoms in the above R¹ and R².

Mₐ^{p+} represents a proton, a metal cation, or an onium cation, and p represents the valence of the cation.

When Mₐ^{p+} represents a metal cation, examples of the metal cation include an alkali metal cation such as a lithium ion, a sodium ion, and a potassium ion, and an alkaline earth metal cation such as a magnesium ion and a calcium ion.

When Mₐ^{p+} represents an onium cation, examples of the onium cation include a trialkylammonium ion, a tetraalkylammonium ion, a tetraalkylphosphonium ion, an imidazolium ion, and an ammonium ion having a spiro skeleton.

p represents the valence of the cation. When Mₐ^{p+} represents a cation with a valence of 1, p is 1, and when Mₐ^{p+} represents a cation with a valence of 2, p is 2.

Mₐ^{p+} may represent a metal cation. When the electrolyte solution containing Component (I) is used in a lithium ion battery application, Mₐ^{p+} is more preferably a lithium ion, and when the electrolyte solution containing Component (I) is used in a sodium ion battery application, Mₐ^{p+} is more preferably a sodium ion.

When R³ represents -R^{a}-S(=O)₂(R^{b}), in the group, R^{a} represents a perfluoroalkylene group having 1 to 4 carbon atoms or a hydrocarbon group having 1 to 10 carbon atoms (the hydrocarbon group may have a hetero atom or a halogen atom, and may have a branched chain or cyclic structure if the number of carbon atoms is 3 or more).

When R^{a} represents a perfluoroalkylene group having 1 to 4 carbon atoms, examples of the perfluoroalkylene group include a difluoromethylene group, a tetrafluoroethylene group, and a hexafluoropropylene group.

When R^{a} represents a hydrocarbon group having 1 to 10 carbon atoms, examples of the hydrocarbon group include an alkylene group having 1 to 10 carbon atoms, an alkenylene group having 2 to 10 carbon atoms, an alkynylene group having 2 to 10 carbon atoms, a cycloalkylene group having 3 to 10 carbon atoms, a cycloalkenylene group having 3 to 10 carbon atoms, an arylene group having 6 to 10 carbon atoms, and a group having 10 carbon atoms or less in which these groups are combined.

Specific examples of an alkylene group having 1 to 10 carbon atoms, an alkenylene group having 2 to 10 carbon atoms, an alkynylene group having 2 to 10 carbon atoms, a cycloalkylene group having 3 to 10 carbon atoms, a cycloalkenylene group having 3 to 10 carbon atoms, and an arylene group having 6 to 10 carbon atoms include a group obtained by removing one hydrogen atom from examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms in the above R¹ and R².

The above hydrocarbon group may have a hetero atom. Examples of the hetero atom include an oxygen atom, a sulfur atom, and a nitrogen atom.

Specific examples of the hydrocarbon group having a hetero atom include the above groups of 1) to 4).

The above hydrocarbon group may have a halogen atom. In other words, any hydrogen atom of the above hydrocarbon group may be substituted with a halogen atom. Examples of the halogen atom include a fluorine atom, a chlorine atom, and an iodine atom, and the halogen atom may be a fluorine atom.

The hydrocarbon group may have a substituent. Examples of the substituent include but not limited to an alkyl group and a halogen atom.

The number of carbon atoms in the hydrocarbon group including a substituent is 1 to 10.

R^{a} may be a perfluoroalkylene group having 1 to 4 carbon atoms, an alkylene group having 1 to 3 carbon atoms, or an arylene group having 6 to 10 carbon atoms, or may be a difluoromethylene group, a methylene group, or a phenylene group.

When R³ represents -R^{a}-S(=O)₂(R^{b}), in the group, R^{b} represents a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₂^{p+})_{q}, M₂^{p+} represents a proton, a metal cation, or an onium cation, and p represents a valence of the cation, q represents a number to satisfy p × q = 1.

When R^{b} represents a halogen atom, examples of the halogen atom include a fluorine atom, a chlorine atom, and an iodine atom.

When R^{b} represents a perfluoroalkyl group having 1 to 4 carbon atoms, examples of the perfluoroalkyl group include a trifluoromethyl group, a pentafluoroethyl group, a heptafluoropropyl group, and a heptafluoroisopropyl group.

When R^{b} represents a hydrocarbon group having 1 to 10 carbon atoms, examples of the hydrocarbon group include an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 10 carbon atoms or less in which these groups are combined.

Specific examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms include examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms in the above R¹ and R².

The above hydrocarbon group may have a hetero atom. Examples of the hetero atom include an oxygen atom, a sulfur atom, and a nitrogen atom.

Specific examples of the hydrocarbon group having a hetero atom include the above groups of 1) to 4).

The above hydrocarbon group may have a halogen atom. In other words, any hydrogen atom of the above hydrocarbon group may be substituted with a halogen atom. Examples of the halogen atom include a fluorine atom, a chlorine atom, and an iodine atom, and a fluorine atom is preferable.

The hydrocarbon group may have a substituent. Examples of the substituent include but not limited to an alkyl group and a halogen atom.

The number of carbon atoms in the hydrocarbon group including a substituent is 1 to 10.

When R^{b} represents an alkoxy group having 1 to 10 carbon atoms, examples of the alkyl group having 1 to 10 carbon atoms in the alkoxy group having 1 to 10 carbon atoms are the same as those of the alkyl group having 1 to 10 carbon atoms in the hydrocarbon group having 1 to 10 carbon atoms in the above R¹ and R².

When R^{b} represents an alkenyloxy group having 2 to 10 carbon atoms, examples of the alkenyl group having 2 to 10 carbon atoms in the alkenyloxy group having 2 to 10 carbon atoms are the same as those of the alkenyl group having 2 to 10 carbon atoms in the hydrocarbon group having 1 to 10 carbon atoms in the above R¹ and R².

When R^{b} represents an alkynyloxy group having 2 to 10 carbon atoms, examples of the alkynyl group having 2 to 10 carbon atoms in the alkynyloxy group having 2 to 10 carbon atoms are the same as those of the alkynyl group having 2 to 10 carbon atoms in the hydrocarbon group having 1 to 10 carbon atoms in the above R¹ and R².

When R^{b} represents a cycloalkoxy group having 3 to 10 carbon atoms, examples of the cycloalkyl group having 3 to 10 carbon atoms in the cycloalkoxy group having 3 to 10 carbon atoms are the same as those of the cycloalkyl group having 3 to 10 carbon atoms in the hydrocarbon group having 1 to 10 carbon atoms in the above R¹ and R².

When R^{b} represents a cycloalkenyloxy group having 3 to 10 carbon atoms, examples of the cycloalkenyl group having 3 to 10 carbon atoms in the cycloalkenyloxy group having 3 to 10 carbon atoms are the same as those of the cycloalkenyl group having 3 to 10 carbon atoms in the hydrocarbon group having 1 to 10 carbon atoms in the above R¹ and R².

When R^{b} represents an aryloxy group having 6 to 10 carbon atoms, examples of the aryl group having 6 to 10 carbon atoms in the aryloxy group having 6 to 10 carbon atoms are the same as those of the aryl group having 6 to 10 carbon atoms in the hydrocarbon group having 1 to 10 carbon atoms in the above R¹ and R².

M₂^{r+} represents a proton, a metal cation, or an onium cation, and r represents the valence of the cation.

When M₂^{r+} represents a metal cation, examples of the metal cation include an alkali metal cation such as a lithium ion, a sodium ion, and a potassium ion, and an alkaline earth metal cation such as a magnesium ion and a calcium ion.

When M₂^{r+} represents an onium cation, examples of the onium cation include a trialkylammonium ion, a tetraalkylammonium ion, a tetraalkylphosphonium ion, an imidazolium ion, and an ammonium ion having a spiro skeleton.

r represents the valence of the cation. When M₂^{r+} represents a cation with a valence of 1, r is 1, and when M₂^{r+} represents a cation with a valence of 2, r is 2.

M₂^{r+} may represent a metal cation. When the electrolyte solution containing Component (I) is used in a lithium ion battery application, M₂^{r+} is more preferably a lithium ion, and when the electrolyte solution containing Component (I) is used in a sodium ion battery application, M₂^{r+} is more preferably a sodium ion.

R^{b} may be a fluorine atom, an alkyl group having 1 to 3 carbon atoms, an alkoxy group having 1 to 3 carbon atoms, or -O⁻(M₂^{r+})₂ (M₂^{r+} represents a metal cation, r represents the valence of the cation. s represents a number to satisfy r × s = 1), or may be a fluorine atom, a methyl group, or -OLi.

When R³ represents -R^{c}-P(=O)(R^{d})(R^{e}), in the group, R^{c} represents a perfluoroalkylene group having 1 to 4 carbon atoms or a hydrocarbon group having 1 to 10 carbon atoms (the hydrocarbon group may have a hetero atom or a halogen atom, and may have a branched chain or cyclic structure if the number of carbon atoms is 3 or more).

Examples of the perfluoroalkylene group having 1 to 4 carbon atoms and the hydrocarbon group having 1 to 10 carbon atoms which are represented by R^{c} include the same groups as those of the above perfluoroalkylene group having 1 to 4 carbon atoms and the hydrocarbon group having 1 to 10 carbon atoms in the above R^{a}.

R^{c} may be a perfluoroalkylene group having 1 to 4 carbon atoms, an alkylene group having 1 to 3 carbon atoms, or an arylene group having 6 to 10 carbon atoms, may be a difluoromethylene group, a methylene group, or a phenylene group, or may be a methylene group.

When R³ represents -R^{c}-P(=O)(R^{d})(R^{e}), in the group, R^{d} and R^{e} each independently represent a halogen atom, a perfluoroalkyl group having 1 to 4 carbon atoms, a hydrocarbon group having 1 to 10 carbon atoms (the hydrocarbon group may have a hetero atom or a halogen atom, and may have a branched chain or cyclic structure if the number of carbon atoms is 3 or more), an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₃^{t+})ᵤ, M₃^{t+} represents a proton, a metal cation, or an onium cation, and t represents a valence of the cation, u represents a number to satisfy t × u = 1.

When R^{d} and R^{e} represent a halogen atom, examples of the halogen atom include a fluorine atom, a chlorine atom, and an iodine atom.

When R^{d} and R^{e} represent a perfluoroalkyl group having 1 to 4 carbon atoms, examples of the perfluoroalkyl group include a trifluoromethyl group, a pentafluoroethyl group, a heptafluoropropyl group, and a heptafluoroisopropyl group.

When R^{d} and R^{e} represent a hydrocarbon group having 1 to 10 carbon atoms, examples of the hydrocarbon group include but not limited to an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 10 carbon atoms or less in which these groups are combined.

Specific examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms include examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms in the above R¹ and R².

The above hydrocarbon group may have a hetero atom. Examples of the hetero atom include an oxygen atom, a sulfur atom, and a nitrogen atom, and specific examples of the hydrocarbon group having a hetero atom include the above groups of 1) to 4).

The above hydrocarbon group may have a halogen atom. In other words, any hydrogen atom of the above hydrocarbon group may be substituted with a halogen atom. Examples of the halogen atom include a fluorine atom, a chlorine atom, and an iodine atom, and the halogen atom may be a fluorine atom.

The hydrocarbon group may have a substituent. Examples of the substituent include but not limited to an alkyl group and a halogen atom.

The number of carbon atoms in the hydrocarbon group including a substituent is 1 to 10.

When R^{d} and R^{e} represent an alkoxy group having 1 to 10 carbon atoms, examples of the alkyl group having 1 to 10 carbon atoms in the alkoxy group having 1 to 10 carbon atoms are the same as those of the alkyl group having 1 to 10 carbon atoms in the hydrocarbon group having 1 to 10 carbon atoms in the above R¹ and R².

When R^{d} and R^{e} represent an alkenyloxy group having 2 to 10 carbon atoms, examples of the alkenyl group having 2 to 10 carbon atoms in the alkenyloxy group having 2 to 10 carbon atoms are the same as those of the alkenyl group having 2 to 10 carbon atoms in the hydrocarbon group having 1 to 10 carbon atoms in the above R¹ and R².

When R^{d} and R^{e} represent an alkynyloxy group having 2 to 10 carbon atoms, examples of the alkynyl group having 2 to 10 carbon atoms in the alkynyloxy group having 2 to 10 carbon atoms are the same as those of the alkynyl group having 2 to 10 carbon atoms in the hydrocarbon group having 1 to 10 carbon atoms in the above R¹ and R².

When R^{d} and R^{e} represent a cycloalkoxy group having 3 to 10 carbon atoms, examples of the cycloalkyl group having 3 to 10 carbon atoms in the cycloalkoxy group having 3 to 10 carbon atoms are the same as those of the cycloalkyl group having 3 to 10 carbon atoms in the hydrocarbon group having 1 to 10 carbon atoms in the above R¹ and R².

When R^{d} and R^{e} represent a cycloalkenyloxy group having 3 to 10 carbon atoms, examples of the cycloalkenyl group having 3 to 10 carbon atoms in the cycloalkenyloxy group having 3 to 10 carbon atoms are the same as those of the cycloalkenyl group having 3 to 10 carbon atoms in the hydrocarbon group having 1 to 10 carbon atoms in the above R¹ and R².

When R^{d} and R^{e} represent an aryloxy group having 6 to 10 carbon atoms, examples of the aryl group having 6 to 10 carbon atoms in the aryloxy group having 6 to 10 carbon atoms are the same as those of the aryl group having 6 to 10 carbon atoms in the hydrocarbon group having 1 to 10 carbon atoms in the above R¹ and R².

M₃^{t+} represents a proton, a metal cation, or an onium cation, and t represents the valence of the cation.

When M₃^{t+} represents a metal cation, examples of the metal cation include an alkali metal cation such as a lithium ion, a sodium ion, and a potassium ion, and an alkaline earth metal cation such as a magnesium ion and a calcium ion.

When M₃^{t+} represents an onium cation, examples of the onium cation include a trialkylammonium ion, a tetraalkylammonium ion, a tetraalkylphosphonium ion, an imidazolium ion, and an ammonium ion having a spiro skeleton.

t represents the valence of the cation. When M₃^{t+} represents a cation with a valence of 1, t is 1, and when M₃^{t+} represents a cation with a valence of 2, t is 2.

M₃^{t+} may represent a metal cation, may be a lithium ion or a sodium ion, or may be a lithium ion.

R^{d} and R^{e} may be an alkoxy group having 1 to 3 carbon atoms, an aryloxy group, or -O⁻(M₃^{t+})ᵤ (M₃^{t+} represents a metal cation, t represents the valence of the cation. u represents the number to satisfy t × u = 1), may be a phenyloxy group, a methoxy group, an ethoxy group, or -OLi, or may be an ethoxy group or -OLi.

R³ may be a halogen atom, a perfluoroalkyl group having 1 to 4 carbon atoms, a cyano group, a hydrocarbon group having 1 to 4 carbon atoms, a group represented by -R^{a}-S(=O)₂(R^{b}) or a group represented by - R^{c}-P(=O)(R^{d})(R^{e}), may be a fluorine atom, a chlorine atom, a trifluoromethyl group, a cyano group, a vinyl group, - CH₂-S(=O)₂OLi, -CH₂-S(=O)₂CH₃, -CH₂-P(=O)(OLi)₂, -CH₂-P(=O)(OCH₃)₂, -CH₂-P(=O)(OCH₂CH₃)₂, may be a fluorine atom, a trifluoromethyl group, a cyano group, or a vinyl group, or may be a fluorine atom, a trifluoromethyl group, or a cyano group.

Specific examples of the compound represented by Formula (1) are shown below, but the present invention is not limited thereto. In the following structural formula, Me represents a methyl group and Et represents an ethyl group.

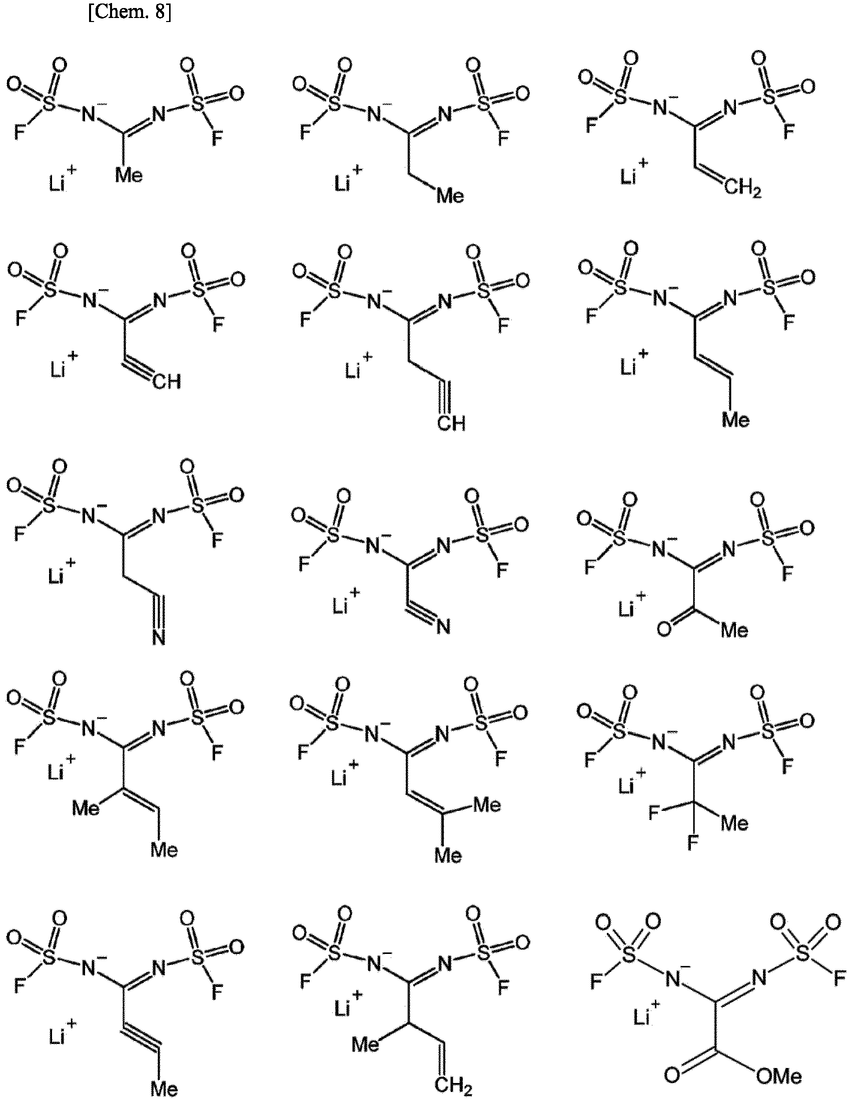

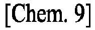

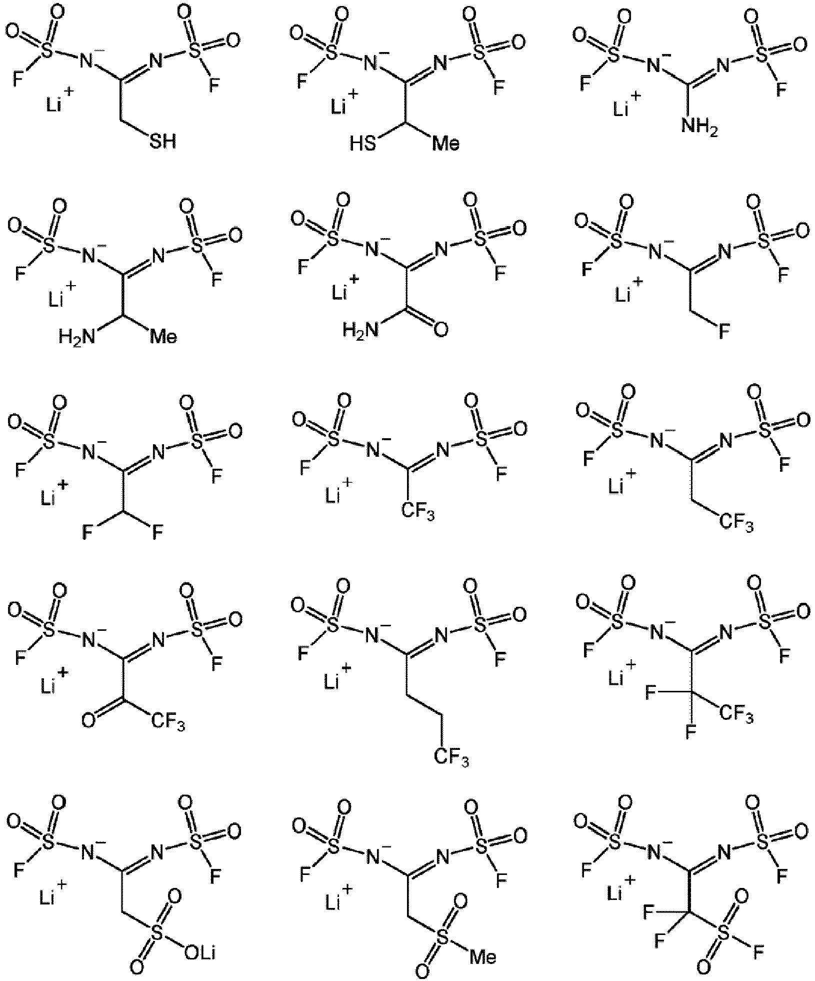

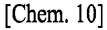

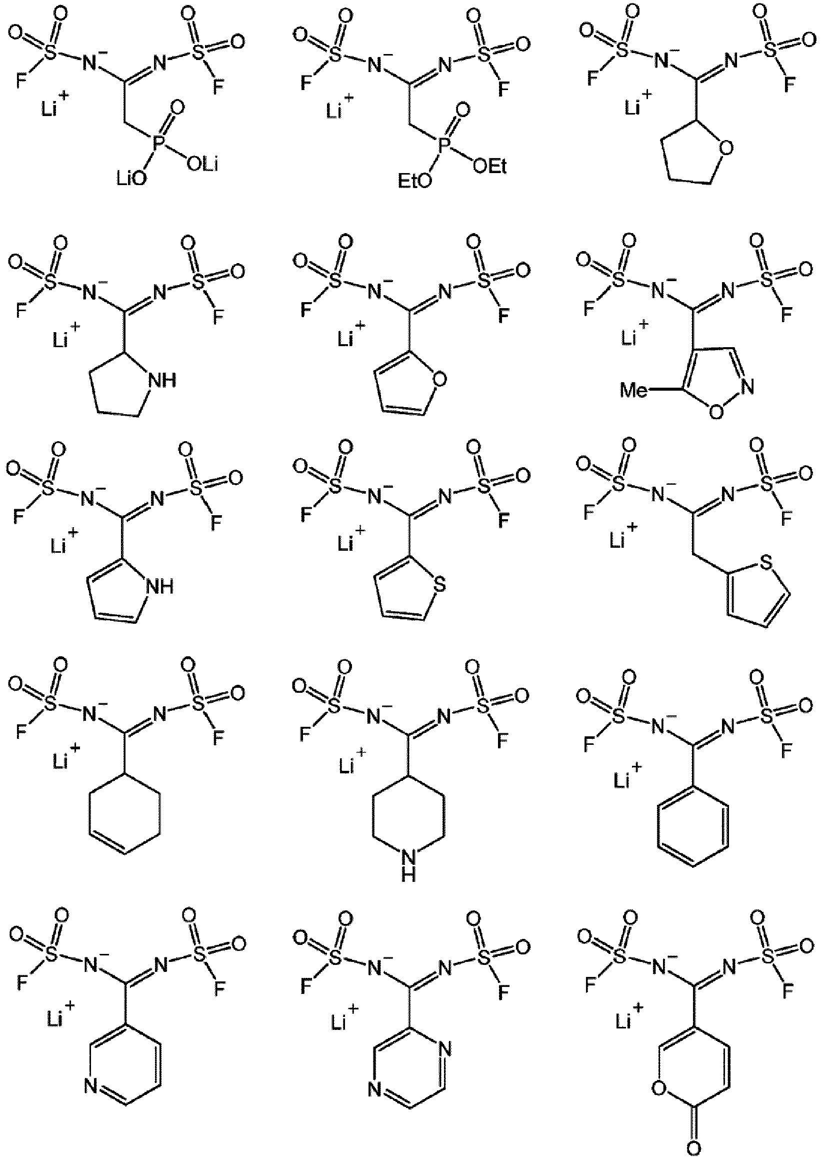

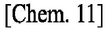

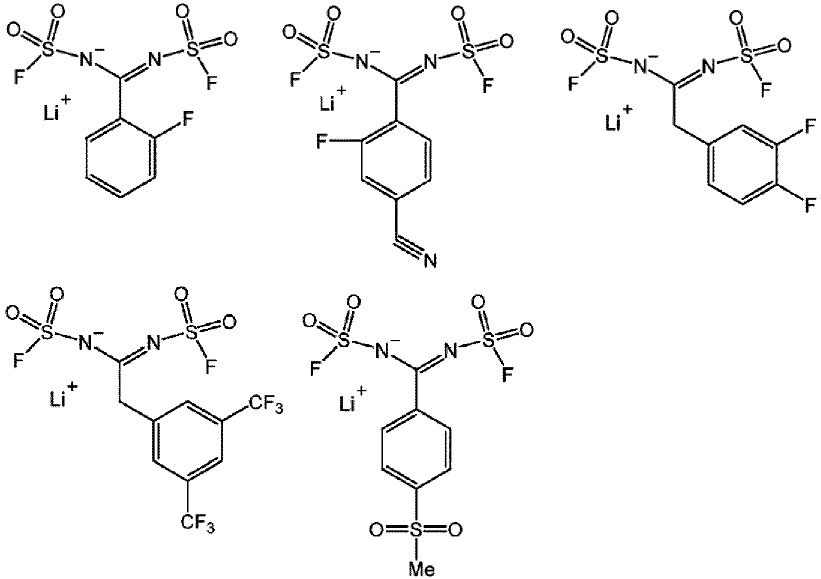

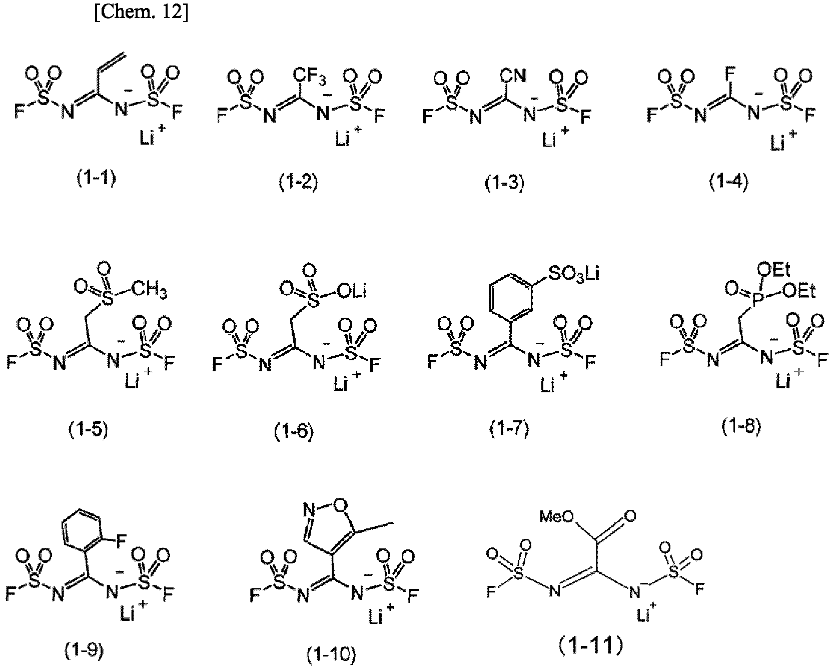

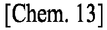

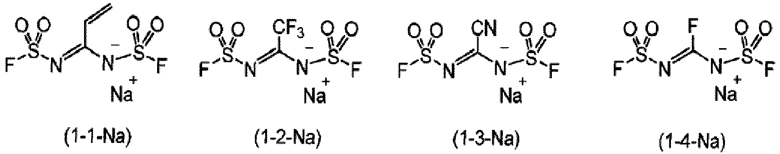

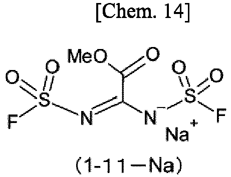

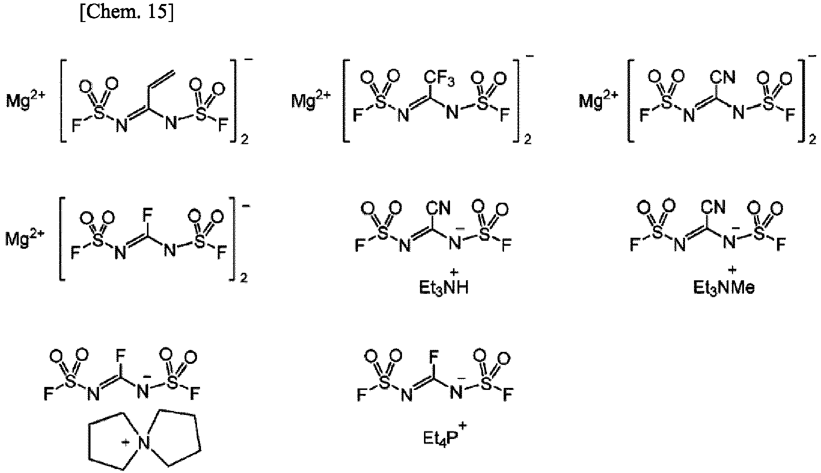

The compound represented by Formula (2) is described below.

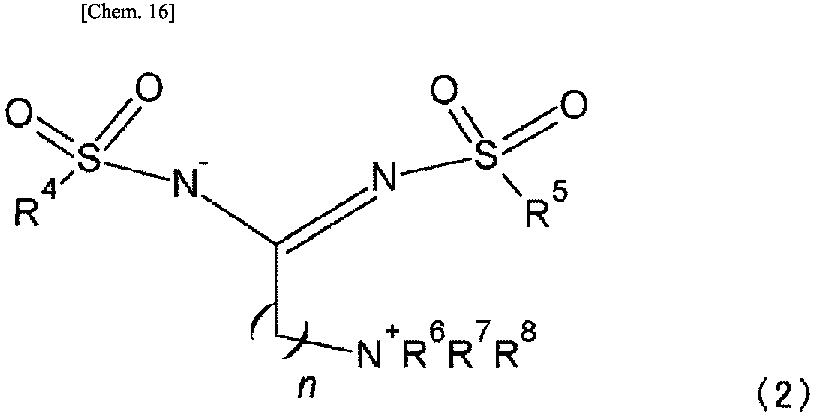

In Formula (2), R⁴ and R⁵ each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).

R⁶ to R⁸ each independently represent a hydrogen atom or a hydrocarbon group having 1 to 10 carbon atoms (the hydrocarbon group may have a hetero atom or a halogen atom, and may have a branched chain or cyclic structure if the number of carbon atoms is 3 or more).

n represents an integer of 1 to 4.

In Formula (2), R⁴ and R⁵ each independently represent a halogen atom, a perfluoroalkyl group having 1 to 4 carbon atoms, or a hydrocarbon group having 1 to 10 carbon atoms.

When R⁴ and R⁵ represent a halogen atom, examples of the halogen atom include a fluorine atom, a chlorine atom, and an iodine atom.

When R⁴ and R⁵ represent a perfluoroalkyl group having 1 to 4 carbon atoms, examples of the perfluoroalkyl group include a trifluoromethyl group, a pentafluoroethyl group, a heptafluoropropyl group, and a heptafluoroisopropyl group.

When R⁴ and R⁵ represent a hydrocarbon group having 1 to 10 carbon atoms, examples of the hydrocarbon group include but not limited to an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 10 carbon atoms or less in which these groups are combined.

Examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are represented by R⁴ and R⁵, include an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are described in the above R¹ and R² in the above Formula (1).

The above hydrocarbon group may have a hetero atom. Examples of the hetero atom include an oxygen atom, a sulfur atom, and a nitrogen atom.

Specific examples of the hydrocarbon group having a hetero atom include the above groups of 1) to 4).

The above hydrocarbon group may have a halogen atom. Specifically, any hydrogen atom of the above hydrocarbon group may be substituted with a halogen atom. Examples of the halogen atom include a fluorine atom, a chlorine atom, and an iodine atom, and the halogen atom may be a fluorine atom.

At least one of R⁴ and R⁵ may represent a halogen atom, at least one thereof may represent a fluorine atom, or both may represent a fluorine atom.

In Formula (2), R⁶ to R⁸ each independently represent a hydrogen atom or a hydrocarbon group having 1 to 10 carbon atoms (the hydrocarbon group may have a hetero atom or a halogen atom, and may have a branched chain or cyclic structure if the number of carbon atoms is 3 or more).

When R⁶ to R⁸ represent a hydrocarbon group having 1 to 10 carbon atoms, examples of the hydrocarbon group include but not limited to an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 10 carbon atoms or less in which these groups are combined.

Examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are represented by R⁶ to R⁸, include an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are described in the above R¹ and R² in the above Formula (1).

The above hydrocarbon group may have a hetero atom. Examples of the hetero atom include an oxygen atom, a sulfur atom, and a nitrogen atom.

Specific examples of the hydrocarbon group having a hetero atom include the above groups of 1) to 4).

The above hydrocarbon group may have a halogen atom. In other words, any hydrogen atom of the above hydrocarbon group may be substituted with a halogen atom. Examples of the halogen atom include a fluorine atom, a chlorine atom, and an iodine atom, and the halogen atom may be a fluorine atom.

R⁶ to R⁸ may be an alkyl group having 1 to 3 carbon atoms, or may be a methyl group.

In Formula (2), n represents an integer of 1 to 4, and may be 1 or 2, or may be 1.

Specific examples of the compound represented by Formula (2) are shown below, but the present invention is not limited thereto.

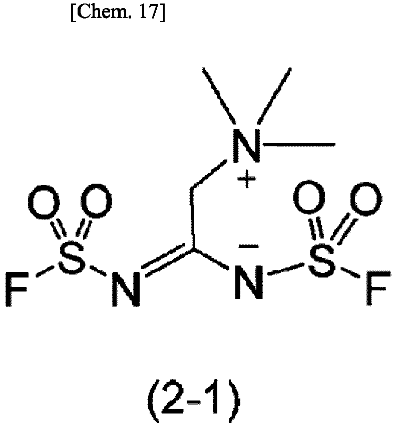

The compound represented by Formula (3) is described below.

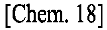

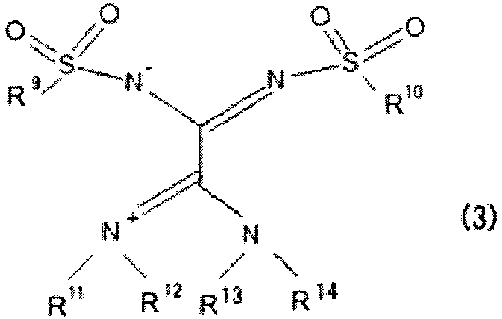

In Formula (3), R⁹ and R¹⁰ each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more).

R¹¹ to R¹⁴ each independently represent a hydrogen atom or a hydrocarbon group having 1 to 10 carbon atoms (the hydrocarbon group may have a hetero atom or a halogen atom, and may have a branched chain or cyclic structure if the number of carbon atoms is 3 or more). R¹² and R¹³ may bond to each other to form a ring.

In Formula (3), R⁹ and R¹⁰ each independently represent a halogen atom, a perfluoroalkyl group having 1 to 4 carbon atoms, or a hydrocarbon group having 1 to 10 carbon atoms.

When R⁹ and R¹⁰ represent a halogen atom, examples of the halogen atom include a fluorine atom, a chlorine atom, and an iodine atom.

When R⁹ and R¹⁰ represent a perfluoroalkyl group having 1 to 4 carbon atoms, examples of the perfluoroalkyl group include a trifluoromethyl group, a pentafluoroethyl group, a heptafluoropropyl group, and a heptafluoroisopropyl group.

When R⁹ and R¹⁰ represent a hydrocarbon group having 1 to 10 carbon atoms, examples of the hydrocarbon group include but not limited to an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 10 carbon atoms or less in which these groups are combined.

Examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are represented by R⁹ and R¹⁰, include an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are described in the above R¹ and R² in the above Formula (1).

The above hydrocarbon group may have a hetero atom. Examples of the hetero atom include an oxygen atom, a sulfur atom, and a nitrogen atom.

Specific examples of the hydrocarbon group having a hetero atom include the above groups of 1) to 4).

The above hydrocarbon group may have a halogen atom. In other words, any hydrogen atom of the above hydrocarbon group may be substituted with a halogen atom. Examples of the halogen atom include a fluorine atom, a chlorine atom, and an iodine atom, and the halogen atom may be a fluorine atom.

At least one of R⁹ and R¹⁰ may represent a halogen atom, at least one thereof may represent a fluorine atom, or both may represent a fluorine atom.

In Formula (3), R¹¹ to R¹⁴ each independently represent a hydrogen atom or a hydrocarbon group having 1 to 10 carbon atoms. R¹² and R¹³ may bond to each other to form a ring.

When R¹¹ to R¹⁴ represent a hydrocarbon group having 1 to 10 carbon atoms, examples of the hydrocarbon group include an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, and a group having 10 carbon atoms or less in which these groups are combined.

Examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are represented by R¹¹ to R¹⁴, include an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are described in the above R¹ and R² in the above Formula (1).

When R¹² and R¹³ bond to each other to form a ring, together with the nitrogen atom bonding to R¹² in Formula (3), the nitrogen atom bonding to R¹³, and a carbon atom bonding to these two nitrogen atoms, a 5-membered ring or a 6-membered ring may be formed, or an imidazole ring may be formed.

R¹¹ to R¹⁴ may be such that R¹¹ and R¹⁴ represent an alkyl group having 1 to 3 carbon atoms, and R¹² and R¹³ bond to each other to form an imidazole ring.

Specific examples of the compound represented by Formula (3) are shown below, but the present invention is not limited thereto.

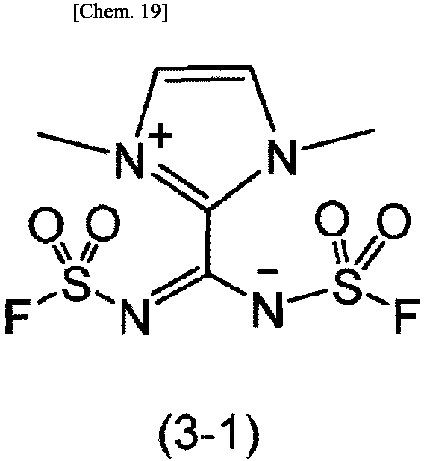

The above Component (I) may be a compound represented by the above Formula (1).

In a nonaqueous electrolyte solution according to the present disclosure, a lower limit of a total amount of the above Component (I) (hereafter, also described as "concentration of (I)") with respect to a total amount (100% by mass) of the nonaqueous electrolyte solution may be 0.01% by mass or more, may be 0.05% by mass or more, and may be 0.1% by mass or more. An upper limit of the concentration of (I) may be 5.0% by mass or less, may be 2.5% by mass or less, or may be 1.8% by mass or less.

Setting the concentration of (I) to 0.01% by mass or more allows for easily exhibiting an effect of suppressing an increase in the initial resistance of the nonaqueous electrolyte solution battery using the nonaqueous electrolyte solution. On the other hand, setting the concentration of (I) to 5.0% by mass or less allows for suppressing an increase in viscosity of the nonaqueous electrolyte solution, and allows for easily exhibiting the effect of suppressing an increase in the initial resistance of the nonaqueous electrolyte solution battery using the nonaqueous electrolyte solution.

In the nonaqueous electrolyte solution according to the present disclosure, one type of compounds in Component (I) may be used alone, or two or more types of compounds may be mixed and used in any combination and any proportion according to an application.

The compound represented by Formula (1) can be produced by various methods. The production method is not limited.

For example, the compound can be obtained by allowing a metal salt of acrylic acid, a metal salt of trifluoroacetic acid, or the like to react with fluorosulfonyl isocyanate or the like.

The compound represented by Formula (2) can be produced by various methods. The production method is not limited.

For example, the compound can be obtained by allowing trimethylglycine or the like to react with fluorosulfonyl isocyanate or the like.

The compound represented by Formula (3) can be produced by various methods. The production method is not limited.

For example, the compound can be obtained by allowing imidazole carboxylic acid or the like to react with fluorosulfonyl isocyanate or the like.

The present disclosure also relates to the compound represented by the above Formula (1), the compound represented by the above Formula (2), and the compound represented by the above Formula (3).

The above compounds are suitably used as an additive in a nonaqueous electrolyte solution.

### <(II) Solute>

The nonaqueous electrolyte solution according to the present disclosure may include (II) a solute (hereinafter, also referred to as "Component (II)").

The solute is not limited, but may be an ionic salt, or may be an ionic salt containing fluorine.

Examples of the solute include an ionic salt containing a pair of at least one cation selected from the group consisting of alkali metal ions such as lithium ions and sodium ions, alkaline earth metal ions, and quaternary ammonium, and at least one anion selected from the group consisting of a hexafluorophosphate anion, a tetrafluoroborate anion, a perchlorate anion, a hexafluoroarsenate anion, a hexafluoroantimonate anion, a trifluoromethanesulfonate anion, a bis(trifluoromethanesulfonyl)imide anion, a bis(pentafluoroethanesulfonyl)imide anion, a (trifluoromethanesulfonyl)(pentafluoroethanesulfonyl)imide anion, a bis(fluorosulfonyl)imide anion, a (trifluoromethanesulfonyl)(fluorosulfonyl)imide anion, a (pentafluoroethanesulfonyl)(fluorosulfonyl)imide anion, and a tris(trifluoromethanesulfonyl)methide anion.

The solute may be at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

One type of these solutes may be used alone, or two or more types thereof may be mixed and used in any combination and any proportion according to an application.

Among the above solutes, from the viewpoint of energy density, output characteristics, life, and the like of a nonaqueous electrolyte solution battery, the cation may be at least one selected from the group consisting of lithium, sodium, potassium, magnesium, and quaternary ammonium, and the anion may be at least one selected from the group consisting of a hexafluorophosphate anion, a tetrafluoroborate anion, a bis(trifluoromethanesulfonyl)imide anion, and a bis(fluorosulfonyl)imide anion.

A total amount of the solute (hereinafter, also described as "solute concentration") in the nonaqueous electrolyte solution according to the present disclosure is not limited, but a lower limit thereof may be 0.5 mol/L or more, 0.7 mol/L or more, or 0.9 mol/L or more. In addition, an upper limit of the solute concentration may be 5.0 mol/L or less, 4.0 mol/L or less, or 2.0 mol/L or less. Setting the solute concentration to 0.5 mol/L or more allows for easily reducing deterioration of the cycle characteristics and the output characteristics of the nonaqueous electrolyte solution battery due to deterioration in ionic conductivity, and setting the solute concentration to 5.0 mol/L or less allows for easily reducing the deterioration in ionic conductivity, and the deterioration in the cycle characteristics and the output characteristics of the nonaqueous electrolyte solution battery due to an increase in the viscosity of the nonaqueous electrolyte solution.

### <(III) Nonaqueous Organic Solvent>

The nonaqueous electrolyte solution according to the present disclosure may include (III) a nonaqueous organic solvent (hereinafter, also referred to as "Component (III)"). A type of the nonaqueous organic solvent is not limited, and any nonaqueous organic solvent can be used.

The nonaqueous organic solvent may be at least one selected from the group consisting of a cyclic ester, a chain ester, a cyclic ether, a chain ether, a sulfone compound, a sulfoxide compound, and an ionic liquid.

Specifically, the nonaqueous organic solvent may be at least one selected from the group consisting of ethyl methyl carbonate (hereinafter, also described as "EMC"), dimethyl carbonate (hereinafter, also described as "DMC"), diethyl carbonate (hereinafter, also described as "DEC"), methyl propyl carbonate, ethyl propyl carbonate, methyl butyl carbonate, 2,2,2-trifluoroethyl methyl carbonate, 2,2,2-trifluoroethyl ethyl carbonate, 2,2,2-trifluoroethyl propyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter also described as "EC"), propylene carbonate (hereinafter, also described as "PC"), butylene carbonate, fluoroethylene carbonate (hereinafter, also described as "FEC"), difluoroethylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethyl sulfoxide, sulfolane, γ-butyrolactone, and γ-valerolactone.

In the present disclosure, an ionic liquid, which has a salt structure, may be used as the nonaqueous organic solvent.

When the nonaqueous organic solvent is at least one selected from the group consisting of a cyclic ester and a chain ester, the solvent achieves excellent input and output characteristics at low temperatures.

When the above nonaqueous organic solvent is at least one selected from the group consisting of cyclic carbonates and chain carbonates, the solvent achieves excellent cycle characteristics at a high temperature.

The nonaqueous organic solvent may contain a cyclic ester, and the cyclic ester may be cyclic carbonate.

Specific examples of the above cyclic carbonate include EC, PC, butylene carbonate, and FEC, and may be at least one selected from the group consisting of EC, PC, and FEC.

The nonaqueous organic solvent may contain a chain ester, and the chain ester may be chain carbonate.

Specific examples of the above chain carbonate include EMC, DMC, DEC, methyl propyl carbonate, ethyl propyl carbonate, 2,2,2-trifluoroethyl methyl carbonate, 2,2,2-trifluoroethyl ethyl carbonate, 1,1,1,3,3,3-hexafluoro-1-propylmethyl carbonate, and 1,1,1,3,3,3-hexafluoro-1-propylethyl carbonate, and may be at least one selected from the group consisting of EMC, DMC, DEC, and methyl propyl carbonate.

Specific examples of the above ester include methyl acetate, ethyl acetate, methyl propionate, ethyl propionate, methyl 2-fluoropropionate, ethyl 2-fluoropropionate, and methyl 3,3,3-trifluoropropionate.

### <Another Additive>

An additive component to be commonly used in the nonaqueous electrolyte solution according to the present disclosure may be further added in any proportion as long as the gist of the present disclosure is not impaired.

Specific examples of another additive include compounds having an overcharge prevention effect, a negative electrode film-forming effect, and a positive electrode protective effect, such as cyclohexylbenzene, cyclohexylfluorobenzene, fluorobenzene, biphenyl, difluoroanisole, tert-butylbenzene, tert-amylbenzene, 2-fluorotoluene, 2-fluorobiphenyl, vinylene carbonate, dimethylvinylene carbonate, vinylethylene carbonate, fluoroethylene carbonate, trans-difluoroethylene carbonate, methyl propargyl carbonate, ethyl propargyl carbonate, dipropargyl carbonate, maleic anhydride, succinic anhydride, propanesultone, 1,3-propanesultone, 1,3-propenesultone, butanesultone, 1,3,2-dioxathiolane-2,2-dioxide, 4-propyl-1,3,2-dioxathiolane-2,2-dioxide, 1,2-ethanedisulfonic anhydride, methylene methanedisulfonate, dimethylene methanedisulfonate, trimethylene methanedisulfonate, methyl methanesulfonate, 1,6-diisocyanatohexane, tris(trimethylsilyl)borate, succinonitrile, (ethoxy)pentafluorocyclotriphosphazene, lithium difluorobis(oxalato)phosphate, sodium difluorobis(oxalato)phosphate, potassium difluorobis(oxalato)phosphate, lithium difluorooxalatoborate, sodium difluorooxalatoborate, potassium difluorooxalatoborate, lithium bis(oxalato)borate, sodium bis(oxalato)borate, potassium bis(oxalato)borate, lithium tetrafluorooxalatophosphate, sodium tetrafluorooxalatophosphate, potassium tetrafluorooxalatophosphate, lithium tris(oxalato)phosphate, sodium tris(oxalato)phosphate, potassium tris(oxalato)phosphate, lithium difluorophosphate, sodium difluorophosphate, potassium difluorophosphate, lithium monofluorophosphate, sodium monofluorophosphate, potassium monofluorophosphate, lithium fluorosulfonate, sodium fluorosulfonate, potassium fluorosulfonate, lithium bis(difluorophosphoryl)imide, sodium bis(difluorophosphoryl)imide, potassium bis(difluorophosphoryl)imide, methanesulfonyl fluoride, ethenesulfonyl fluoride, and phenyl difluorophosphate.

The nonaqueous electrolyte solution according to the present disclosure may contain at least one selected from vinylene carbonate, bis(oxalato)borate, difluorooxalatoborate, difluorobis(oxalato)phosphate, tetrafluorooxalatophosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene. A content of the above additive in the nonaqueous electrolyte solution may be 0.01% by mass or more and 5.0% by mass or less with respect to the total amount of the nonaqueous electrolyte solution.

The nonaqueous electrolyte solution according to the present disclosure may contain a compound represented by the following Formula (4) as another additive.

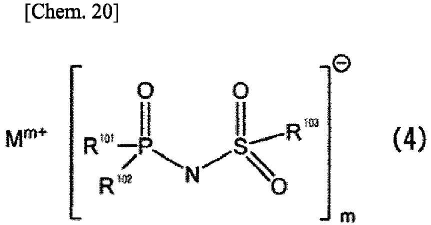

[In Formula (4), R¹⁰¹ to R¹⁰³ are each independently a fluorine atom or an organic group selected from a linear alkyl group having 1 to 10 carbon atoms, a branched alkyl group having 3 to 10 carbon atoms, a linear alkoxy group having 1 to 10 carbon atoms, a branched alkoxy group having 3 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 10 carbon atoms, a cycloalkoxy group having 3 to 10 carbon atoms, a cycloalkenyl group having 3 to 10 carbon atoms, a cycloalkenyloxy group having 3 to 10 carbon atoms, an aryl group having 6 to 10 carbon atoms, and an aryloxy group having 6 to 10 carbon atoms, and the organic group may have a fluorine atom, an oxygen atom, or an unsaturated bond. Here, at least one of R¹⁰¹ to R¹⁰³ is a fluorine atom.

M^{m+} represents an alkali metal cation, an alkaline earth metal cation, or an onium cation, and m represents the same integer as a valence of the corresponding cation.]

When the compound (salt having an imide anion) represented by Formula (4) has at least one P-F bond or S-F bond, the compound achieves excellent low-temperature characteristics. The larger number of P-F bonds and S-F bonds in the salt having the above imide anion equates to the more improvement in low-temperature characteristics, and in the salt having the imide anion represented by the above Formula (4), the compound represented by Formula (4) may be a compound in which all of R¹⁰¹ to R¹⁰³ are fluorine atoms.

In the salt having the imide anion represented by the above Formula (4), the preferable compound is a compound in which at least one of R¹⁰¹ to R¹⁰³ is a fluorine atom, and at least one of R¹⁰¹ to R¹⁰³ is selected from a hydrocarbon group having 6 or less carbon atoms and optionally having a fluorine atom.

In the salt having the imide anion represented by the above Formula (4), the compound represented by Formula (4) may be a compound in which at least one of R¹⁰¹ to R¹⁰³ is a fluorine atom, and at least one of R¹⁰¹ to R¹⁰³ is selected from a methyl group, a methoxy group, an ethyl group, an ethoxy group, a propyl group, a propoxy group, a vinyl group, an allyl group, an allyloxy group, an ethynyl group, a 2-propynyl group, a 2-propynyloxy group, a phenyl group, a phenyloxy group, a 2,2-difluoroethyl group, a 2,2-difluoroethyloxy group, a 2,2,2-trifluoroethyl group, a 2,2,2-trifluoroethyloxy group, a 2,2,3,3-tetrafluoropropyl group, a 2,2,3,3-tetrafluoropropyloxy group, a 1,1,1,3,3,3-hexafluoroisopropyl group, and a 1,1,1,3,3,3-hexafluoroisopropyloxy group.

A counter cation M^{m+} of the salt having the imide anion represented by Formula (4) may be selected from the group consisting of lithium ion, sodium ion, potassium ion, and tetraalkylammonium ion.

In the above Formula (4), examples of the alkyl group and the alkoxy group represented by R¹⁰¹ to R¹⁰³ include an alkyl group having 1 to 10 carbon atoms, a fluorine-containing alkyl group, and an alkoxy group derived from these groups, such as a methyl group, an ethyl group, a propyl group, an isopropyl group, a butyl group, a sec-butyl group, a tert-butyl group, a pentyl group, a 2,2-difluoroethyl group, 2,2,2-trifluoroethyl group, 2,2,3,3-tetrafluoropropyl group, and a 1,1,1,3,3,3-hexafluoroisopropyl group.

Examples of the alkenyl group and the alkenyloxy group include an alkenyl group having 2 to 10 carbon atoms such as a vinyl group, an allyl group, a 1-propenyl group, an isopropenyl group, a 2-butenyl group, and a 1,3-butadienyl group, fluorine-containing alkenyl groups, and alkenyloxy groups derived from these groups.

Examples of the alkynyl group and the alkynyloxy group include an alkynyl group having 2 to 10 carbon atoms such as an ethynyl group, a 2-propynyl group, and a 1,1-dimethyl-2-propynyl group, fluorine-containing alkynyl groups, and alkynyloxy groups derived from these groups.

Examples of the cycloalkyl group and the cycloalkoxy group include a cycloalkyl group having 3 to 10 carbon atoms such as a cyclopentyl group and a cyclohexyl group, fluorine-containing cycloalkyl groups, and cycloalkoxy groups derived from these groups.

Examples of the cycloalkenyl group and the cycloalkenyloxy group include a cycloalkenyl group having 3 to 10 carbon atoms such as a cyclopentenyl group and a cyclohexenyl group, fluorine-containing cycloalkenyl groups, and cycloalkenyloxy groups derived from these groups.

Examples of the aryl group and the aryloxy group include an aryl group having 6 to 10 carbon atoms such as a phenyl group, a tolyl group, and an xylyl group, fluorine-containing aryl groups, and aryloxy groups derived from these groups.

Specific examples and synthesis methods of the salt having the imide anion represented by Formula (4) include those described in WO2017/111143.

A content of another additive (salt having an imide anion represented by the above Formula (4)) in the nonaqueous electrolyte solution may be 0.01% by mass or more and 8.0% by mass or less with respect to the total amount of the nonaqueous electrolyte solution.

When the content of the ionic salt exemplified as the solute is less than 0.5 mol/L, in the nonaqueous electrolyte solution, the ionic salt can exert a negative electrode film-forming effect and/or a positive electrode protective effect as "another additive". In this case, the content in the nonaqueous electrolyte solution may be 0.01% by mass to 5.0% by mass.

For example, when the nonaqueous electrolyte solution battery is a lithium ion battery, examples of the ionic salt include lithium hexafluorophosphate, lithium tetrafluoroborate, lithium trifluoromethanesulfonate, lithium bis(trifluoromethanesulfonyl)imide, lithium bis(fluorosulfonyl)imide, and lithium (trifluoromethanesulfonyl)(fluorosulfonyl)imide, and when the nonaqueous electrolyte solution battery is a sodium ion battery, examples of the ionic salt include sodium hexafluorophosphate, sodium tetrafluoroborate, sodium trifluoromethanesulfonate, sodium bis(trifluoromethanesulfonyl)imide, sodium bis(fluorosulfonyl)imide, and sodium (trifluoromethanesulfonyl)(fluorosulfonyl)imide.

An alkali metal salt other than the above solutes may be used as an additive.

Specific examples include carboxylates such as lithium acrylate, sodium acrylate, lithium methacrylate, and sodium methacrylate, and sulfuric acid ester salts such as lithium methyl sulfate, sodium methyl sulfate, lithium ethyl sulfate, and sodium ethyl sulfate.

From a viewpoint of improving durability (life) of a battery, when the nonaqueous electrolyte solution battery is a lithium ion battery, the nonaqueous electrolyte solution according to the present disclosure may contain, with respect to the total amount of the nonaqueous electrolyte solution, 0.01% by mass to 5.0% by mass of at least one selected from vinylene carbonate, fluoroethylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, lithium (difluorophosphoryl)(fluorosulfonyl)imide, lithium difluorophosphate, lithium fluorosulfonate, 1,3-propenesultone, 1,3-propanesultone, 1,3,2-dioxathiolane-2,2-dioxide, 1,2-ethanedisulfonic anhydride, and 4-propyl-1,3,2-dioxathiolane-2,2-dioxide in the above another additive.

When the nonaqueous electrolyte solution battery is a sodium ion battery, the nonaqueous electrolyte solution according to the present disclosure may contain, with respect to the total amount of the nonaqueous electrolyte solution, 0.01% by mass to 5.0% by mass of at least one selected from vinylene carbonate, fluoroethylene carbonate, sodium bis(oxalato)borate, sodium difluorooxalatoborate, sodium difluorobis(oxalato)phosphate, sodium tetrafluorooxalatophosphate, sodium bis(fluorosulfonyl)imide, sodium (difluorophosphoryl)(fluorosulfonyl)imide, sodium difluorophosphate, sodium fluorosulfonate, 1,3-propenesultone, 1,3-propanesultone, 1,3,2-dioxathiolane-2,2-dioxide, 1,2-ethanedisulfonic anhydride, and 4-propyl-1,3,2-dioxathiolane-2,2-dioxide.

In addition, the nonaqueous electrolyte solution according to the present disclosure can also contain a polymer, and as in the case of being used in a nonaqueous electrolyte solution battery referred to as a polymer battery, the nonaqueous electrolyte solution can be quasi-solidified with a gelling agent or a cross-linked polymer. A polymer solid electrolyte includes one containing the nonaqueous organic solvent as a plasticizer.

The above polymer is not limited as long as the polymer is an aprotic polymer capable of dissolving the compound represented by the above Formula (1), Formula (2) or Formula (3), the above solute, and the above another additive. Examples of the above polymer include polymers having polyethylene oxide as a main chain or a side chain, homopolymers or copolymers of polyvinylidene fluoride, methacrylic acid ester polymers, and polyacrylonitrile. When a plasticizer is added to these polymers, an aprotic nonaqueous organic solvent may be added among the above nonaqueous organic solvents.

### [2. Nonaqueous Electrolyte Solution Battery]

The nonaqueous electrolyte solution battery according to the present disclosure includes, at least, the nonaqueous electrolyte solution according to the present disclosure described above, a negative electrode, a positive electrode, and a separator. An exterior body or the like may be further included.

The negative electrode is not limited, but may use a material capable of reversibly intercalating and deintercalating alkali metal ions such as lithium ions and sodium ions, or alkaline earth metal ions.

For example, in the case of the lithium ion secondary battery in which cations are mainly lithium, a negative electrode active material constituting the negative electrode is capable of doping and dedoping lithium ions. Examples of the negative electrode active material includes a material containing at least one selected from, for example, a carbon material in which a d value of a lattice plane (002 plane) in X-ray diffraction is 0.340 nm or less, a carbon material in which the d value of the lattice plane (002 plane) in X-ray diffraction exceeds 0.340 nm, oxides of one or more metal selected from Si, Sn, and Al, one or more metals selected from Si, Sn, and Al, alloys containing these metals, alloys of these metals and lithium, or alloys of the above alloys and lithium, and lithium titanium oxide. One of these negative electrode active materials can be used alone, or two or more thereof can be used in combination. Lithium metal, metal nitrides, tin compounds, conductive polymers, and the like may also be used.

For example, in a case of a sodium ion secondary battery in which cations are mainly sodium, as the negative electrode active material constituting the negative electrode, sodium metal, an alloy of sodium metal and other metals such as tin, an intermetallic compound, various carbon materials such as hard carbon, a metal oxide such as titanium oxide, a metal nitride, (elemental) tin, a tin compound, activated carbon, a conductive polymer, and the like are used. In addition to these, (elemental) phosphorus such as red phosphorus and black phosphorus, phosphorus compounds such as Co-P, Cu-P, Sn-P, Ge-P, and Mo-P, (elemental) antimony, antimony compounds such as Sb/C and Bi-Sb, and the like are used. One type of these negative electrode active materials may be used alone, or two or more types thereof may be used in combination.

The positive electrode is not limited, but may use a material capable of reversibly intercalating and deintercalating alkali metal ions such as lithium ions and sodium ions, or alkaline earth metal ions.

For example, when the cation is lithium, lithium-containing transition metal composite oxides such as LiCoOz, LiNiOz, LiMnOz, and LiMn₂O₄, these lithium-containing transition metal composite oxides including a mixture of a plurality of transition metals such as Co, Mn, and Ni, these lithium-containing transition metal composite oxides in which a part of the transition metals is substituted by a metal other than the transition metals, phosphate compounds of transition metals such as LiFePO₄, LiCoPO₄, and LiMnPO₄ referred to as olivine, oxides such as TiOz, V₂O₅, and MoO₃, sulfides such as TiSz and FeS, conductive polymers such as polyacetylene, polyparaphenylene, polyaniline, and polypyrrole, activated carbon, radical-generating polymers, carbon materials, and the like may be used as a positive electrode material.

For example, when the cation is sodium, sodium-containing transition metal composite oxides such as NaCrO₂, NaFe_{0.5}Co_{0.5}O₂, NaFe_{0.4}Mn_{0.3}Ni_{0.3}O₂, NaNi_{0.5}Ti_{0.3}Mn_{0.2}O₂, NaNi_{1/3}Ti_{1/3}Mn_{1/3}O₂, NaNi_{0.33}Ti_{0.33}Mn_{0.16}Mg_{0.17}O₂, Na_{2/3}Ni_{1/3}Ti_{1/6}Mn_{1/2}O₂, and Na_{2/3}Ni_{1/3}Mn_{2/3}O₂, these sodium-containing transition metal composite oxides including a mixture of a plurality of transition metals such as Co, Mn, and Ni, these sodium-containing transition metal composite oxides in which a part of the transition metals is substituted with a metal other than the transition metals, polyanion type compounds such as NaFePO₄, NaVPO₄F, Na₃V₂(PO₄)₃, and Na₂Fe₂(SO₄)₃, sodium salts of a Prussian Blue analogues represented by a composition formula NaₐM_{b}[Fe(CN)₆]_{c} (M = Cr, Mn, Fe, Co, Ni, Cu, or Zn, 0 ≤ a ≤ 2, 0.5 ≤ b ≤ 1.5, 0.5 ≤ c ≤ 1.5), oxides such as TiO₂, V₂O₅, and MoO₃, sulfides such as TiS₂ and FeS, conductive polymers such as polyacetylene, polyparaphenylene, polyaniline, and polypyrrole, activated carbon, radical-generating polymers, carbon materials, and the like may be used as a positive electrode material (positive electrode active material).

Acetylene black, ketjen black, carbon fiber, or graphite as a conductive material, and polytetrafluoroethylene, polyvinylidene fluoride, SBR resin, or the like as a binder may be added to the positive electrode material and negative electrode material. An electrode sheet molded into a sheet shape may be used.

As a separator for preventing contact between the positive electrode and the negative electrode, a nonwoven fabric or porous sheet made of polyolefin, polyethylene, paper, glass fiber, or the like may be used.

An electrochemical device having a shape such as a coin shape, a cylindrical shape, a square shape, or an aluminum laminate sheet shape is assembled based on the above elements.

### EXAMPLE

The present disclosure will be described in more detail below with reference to Examples, but the present disclosure is not limited by these descriptions.

### <Synthesis Example 1-1>

### Synthesis of Compound (1-1)

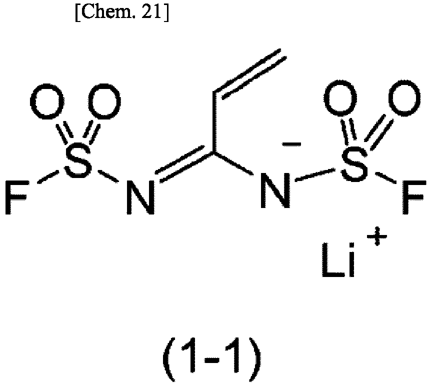

After 30 g of acetonitrile (MeCN) and 0.8 g of acrylic acid Li salt were put into a 50 ml eggplant flask, 2.8 g of fluorosulfonyl isocyanate was slowly added. After stirring at 40°C or lower for 1 hour, concentration was carried out to obtain 2.3 g (yield: 95%) of Compound (1-1). ¹H NMR(CD₃CN) σ_{H} 6.84 ppm, 6.34 ppm, 5.78 ppm, ¹⁹F NMR(CD₃CN) σ_{F} 52.8 ppm.

### <Synthesis Example 1-2>

### Synthesis of Compound (1-2)

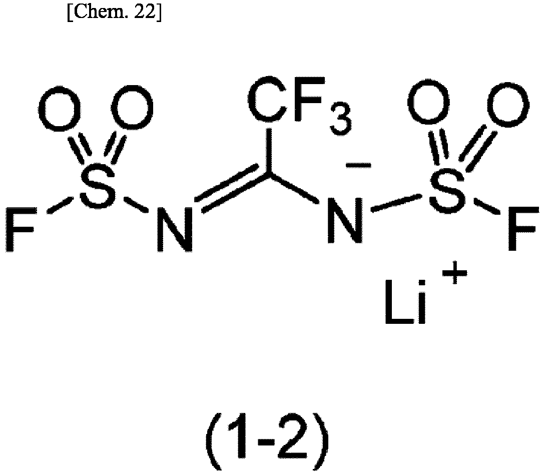

After 30 g of MeCN and 1.8 g of trifluoroacetic acid Li salt were put into the 50 ml eggplant flask, 4.6 g of chlorosulfonyl isocyanate was slowly added. After stirring at 40°C or lower for 15 hours, 1.3 g of sodium fluoride was added, and the mixture was stirred at 40°C or lower for 15 hours. The reaction solution was filtered, and the filtrate was concentrated to obtain 3.2 g of Compound (1-2) ((yield: 76%). ¹⁹F NMR (CD₃CN) σ_{F} 53.9 ppm, -67.8 ppm.

### <Synthesis Example 1-3>

### Synthesis of Compound (1-3)

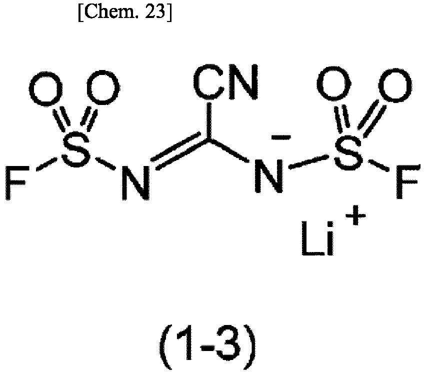

After 30 g of MeCN and 0.7 g of lithium cyanide were put into the 50 ml eggplant flask, 5.7 g of fluorosulfonyl isocyanate was slowly added. After stirring at 40°C or lower for 1 hour, concentration was carried out to obtain 3.9 g (yield: 77%) of Compound (1-3). ¹⁹F NMR (CD₃CN) or 52.2 ppm.

### <Synthesis Example 1-4>

### Synthesis of Compound (1-4)

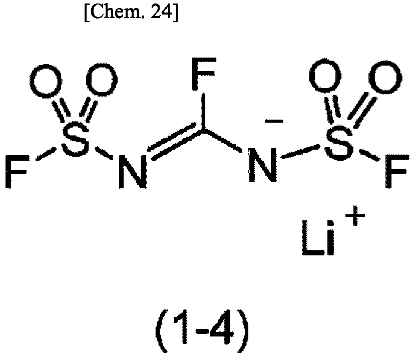

After 30 g of MeCN and 1.16 g of potassium fluoride were put into the 50 ml eggplant flask, 6.0 g of fluorosulfonyl isocyanate was slowly added. After stirring at 40°C or lower for 2 hours, 0.84 g of lithium chloride was added, and the mixture was stirred at a room temperature (23°C) for 2 hours. The reaction solution was filtered, and the filtrate was concentrated to obtain 3.7 g of Compound (1-4) (yield: 80%). ¹⁹F NMR (CD₃CN) σ_{F} 49.4 ppm, - 16.0 ppm.

### <Synthesis Example 1-5>

### Synthesis of Compound (1-5)

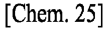

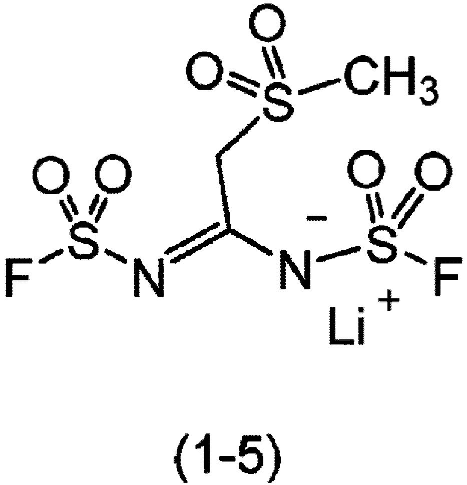

After 30 g of MeCN and 1.60 g of lithium methanesulfonylacetate were put into the 50 ml eggplant flask, 3.4 g of fluorosulfonyl isocyanate was slowly added. After stirring at 40°C or lower for 1 hour, concentration was carried out to obtain 2.9 g (yield: 83%) of Compound (1-5). ¹H NMR(CD₃CN) σ_{H} 3.10, 4.45 ppm, ¹⁹F NMR(CD₃CN) σ_{F} 53.2 ppm.

### <Synthesis Example 1-6>

### Synthesis of Compound (1-6)

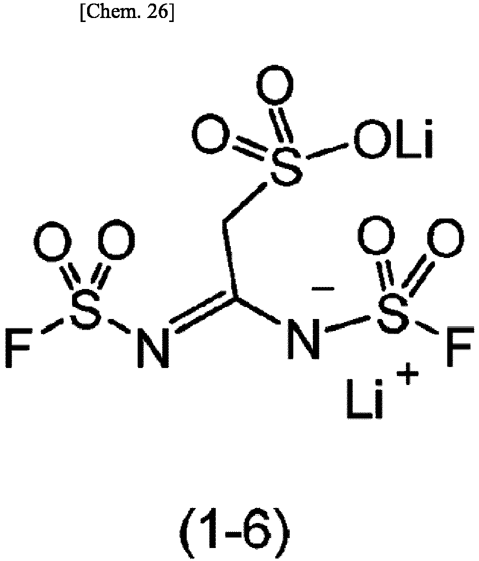

After 30 g of MeCN and 1.5 g of lithium sulfoacetate were put into the 50 ml eggplant flask, 3.0 g of fluorosulfonyl isocyanate was slowly added. After stirring at 40°C or lower for 1 hour, concentration was carried out to obtain 2.4 g (yield: 77%) of Compound (1-6). ¹H NMR(CD₃CN) σ_{H} 4.55 ppm, ¹⁹F NMR(CD₃CN) σ_{F} 53.0 ppm.

### <Synthesis Example 1-7>

### Synthesis of Compound (1-7)

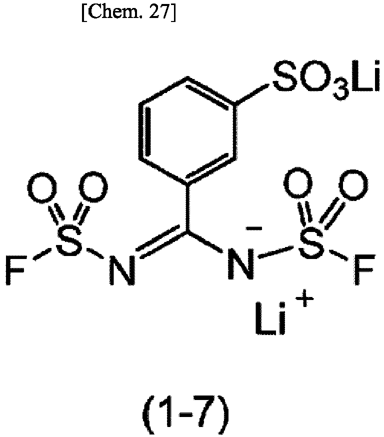

After 30 g of MeCN and 2.1 g of lithium sulfobenzoate were put into the 50 ml eggplant flask, 3.0 g of fluorosulfonyl isocyanate was slowly added. After stirring at 40°C or lower for 1 hour, concentration was carried out to obtain 2.8 g (yield: 74%) of Compound (1-7). ¹H NMR(CD₃CN) σ_{H} 7.33 ppm to 7.92 ppm, ¹⁹F NMR(CD₃CN) σ_{F} 53.5 ppm.

### <Synthesis Example 1-8>

### Synthesis of Compound (1-8)

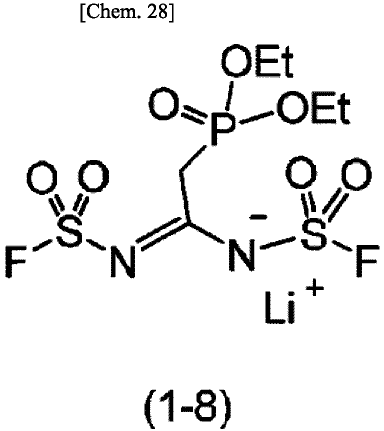

After 30 g of MeCN and 3.1 g of diethylphosphonoacetic acid were put into the 50 ml eggplant flask, 4.8 g of fluorosulfonyl isocyanate was slowly added. After stirring at 40°C or lower for 1 hour, concentration was carried out to obtain 4.4 g (yield: 75%) of Compound (1-8). ¹H NMR(CD₃CN) σ_{H} 4.11 ppm, 3.43 ppm, 1.30 ppm, ¹⁹F NMR(CD₃CN) σ_{F} 52.4 ppm.

### <Synthesis Example 1-9>

### Synthesis of Compound (1-9)

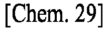

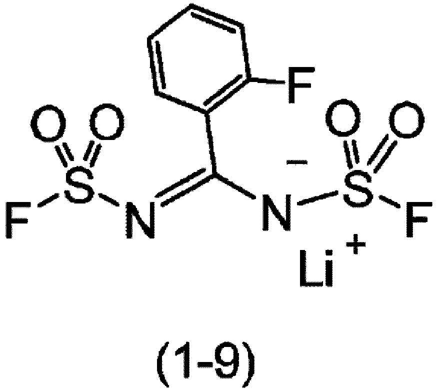

After 30 g of MeCN and 1.5 g of lithium fluorobenzoate were put into the 50 ml eggplant flask, 3.2 g of fluorosulfonyl isocyanate was slowly added. After stirring at 40°C or lower for 1 hour, concentration was carried out to obtain 2.5 g (yield: 81%) of Compound (1-9). ¹H NMR(CD₃CN) σ_{H} 7.11 ppm to 7.48 ppm, ¹⁹F NMR(CD₃CN) σ_{F} 52.9 ppm.

### <Synthesis Example 1-10>

### Synthesis of Compound (1-10)

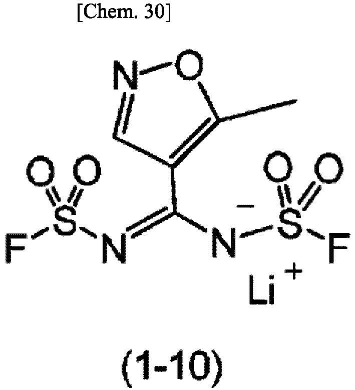

After 30 g of MeCN and 1.3 g of lithium 5-methyl-isoxazole-4-carboxylate were put into the 50 ml eggplant flask, 3.0 g of fluorosulfonyl isocyanate was slowly added. After stirring at 40°C or lower for 1 hour, concentration was carried out to obtain 2.7 g (yield: 93%) of Compound (1-10). ¹H NMR(CD₃CN) σ_{H} 2.57 ppm, 8.59 ppm, ¹⁹F NMR(CD₃CN) σ_{F} 53.4 ppm.

### <Synthesis Example 1-11>

### Synthesis of Compound (1-11)

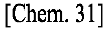

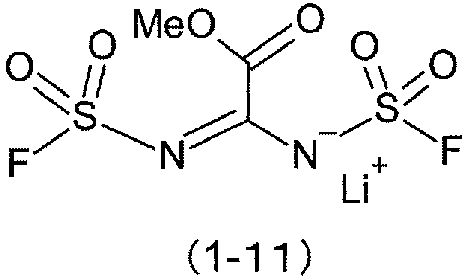

After 30 g of MeCN and 1.1 g of lithium methyl oxalate were put into the 50 ml eggplant flask, 3.0 g of fluorosulfonyl isocyanate was slowly added. After stirring at 40°C or lower for 1 hour, concentration was carried out to obtain 2.4 g (yield: 90%) of Compound (1-11). ¹H NMR(CD₃CN) σ_{H} 3.81 ppm, ¹⁹F NMR(CD₃CN) σ_{F} 52.4 ppm.

### <Synthesis Example 2-1>

### Synthesis of Compound (2-1)

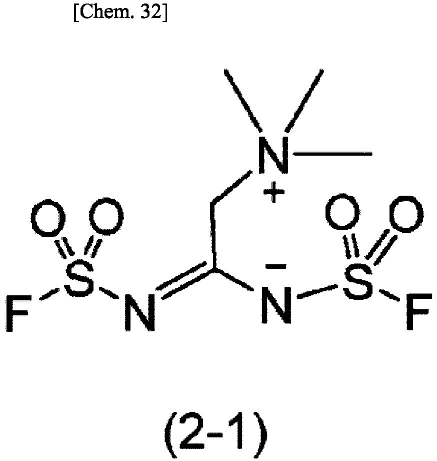

After 30 g of MeCN and 1.2 g of trimethylglycine were put into the 50 ml eggplant flask, 3.0 g of fluorosulfonyl isocyanate was slowly added. After stirring at 40°C or lower for 1 hour, concentration was carried out to obtain 2.7 g (yield: 95%) of Compound (2-1). ¹H NMR(CD₃CN) σ_{H} 3.28 ppm 4.48 ppm, ¹⁹F NMR(CD₃CN) σ_{F} 54.1 ppm.

### <Synthesis Example 3-1>

### Synthesis of Compound (3-1)

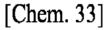

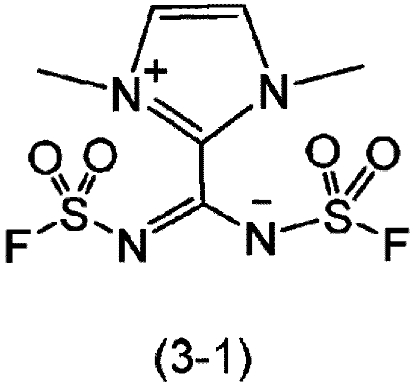

After 30 g of MeCN and 2.1 g of imidazole carboxylic acid were put into the 50 ml eggplant flask, 4.5 g of fluorosulfonyl isocyanate was slowly added. After stirring at 40°C or lower for 1 hour, concentration was carried out to obtain 3.6 g (yield: 80%) of Compound (3-1). ¹H NMR(CD₃CN) σ_{H} 3.85, 7.47 ppm, ¹⁹F NMR(CD₃CN) σ_{F} 54.0 ppm.

The following Compounds (1-1-Na) to (1-4-Na) and (1-11-Na) were obtained by subjecting the above Compounds (1-1) to (1-4) and (1-11) to a cation exchange reaction, respectively.

Compounds (1-1-Na) to (1-4-Na) and (1-11-Na) can also be obtained by using each sodium carboxylate (however, in the case of (1-4-Na), sodium fluoride) corresponding to the raw material and allowing it to react with fluorosulfonyl isocyanate.

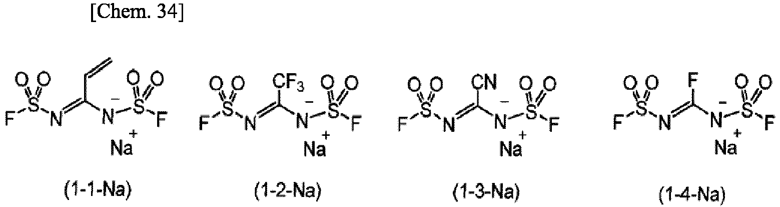

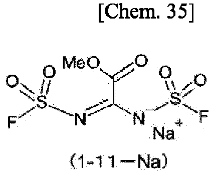

### [Preparation of Nonaqueous Electrolyte Solutions according to Examples and Comparative Examples]

### <Comparative Example 1-1>

### (Preparation of LiPF₆ Solution)

EC, EMC, and DMC were mixed in a volume ratio of EC:EMC:DMC = 25:50:25 in a glove box with a dew point of -60°C or lower (Component (III)). After that, LiPF₆ (Component (II)) was added such that LiPF₆ had a concentration of 1.0 mol/L, with an internal temperature at 40°C or lower kept. The mixture was stirred, and LiPF₆ was completely dissolved to obtain a LiPF₆ solution. This LiPF₆ solution was set as Comparative Nonaqueous Electrolyte Solution 1-1.

### <Example 1-1>

### (Preparation of Nonaqueous Electrolyte Solution 1-1)

EC, EMC, and DMC were mixed in a volume ratio of EC:EMC:DMC = 25:50:25 in a glove box with a dew point of -60°C or lower (Component (III)). After that, LiPF₆ (Component (II)) was added such that LiPF₆ had a concentration of 1.0 mol/L, with the internal temperature at 40°C or lower kept, Compound (1-1) (Component (I)) corresponding to the compound represented by Formula (1) was added such that Compound (1-1) had a concentration of 0.05% by mass with respect to the total amount of the nonaqueous electrolyte solution. The mixture was stirred for 1 hour and Compound (1-1) was dissolved to obtain Nonaqueous Electrolyte Solution 1-1 according to Example 1-1.

### <Examples 1-2 to 1-18, Comparative Example 1-2>

### (Preparation of Nonaqueous Electrolyte Solutions 1-2 to 1-18 and Comparative Nonaqueous Electrolyte Solution 1-2)

Except that a type and content of Component (I) (or comparative compound) were changed as described in Table 1, Nonaqueous Electrolyte Solutions 1-2 to 1-18 and Comparative Nonaqueous Electrolyte Solution 1-2 were obtained in the same way as in the preparation of Nonaqueous Electrolyte Solution 1-1. Compound (X) was synthesized by the same method as the preparation method disclosed in WO2021/015264.

The structure of Compound (X) used in Comparative Examples is shown below.

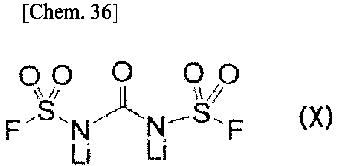

### <Examples 2-1 to 2-6, Comparative Examples 2-1 to 2-2>

### (Preparation of Nonaqueous Electrolyte Solutions 2-1 to 2-6 and Comparative Nonaqueous Electrolyte Solutions 2-1 to 2-2)

Furthermore, except that vinylene carbonate (VC) as another additive was added and dissolved such that VC had a concentration described in Table 2, Nonaqueous Electrolyte Solutions 2-1 to 2-6 and Comparative Nonaqueous Electrolyte Solutions 2-1 to 2-2 were respectively obtained in the same way as in the preparation of Nonaqueous Electrolyte Solutions 1-4, 1-8, 1-10, 1-12, 1-14, and 1-16, and Comparative Nonaqueous Electrolyte Solutions 1-1 to 1-2.

### <Examples 3-1 to 3-6, Comparative Examples 3-1 to 3-2>

### (Preparation of Nonaqueous Electrolyte Solutions 3-1 to 3-6 and Comparative Nonaqueous Electrolyte Solutions 3-1 to 3-2)

Except that VC was changed to lithium bis(oxalato)borate (BOB), and a content of Component (I) (or comparative compound) was changed as described in Table 3, Nonaqueous Electrolyte Solutions 3-1 to 3-6 and Comparative Nonaqueous Electrolyte Solutions 3-1 to 3-2 were respectively obtained in the same way as in the preparation of Nonaqueous Electrolyte Solutions 2-1 to 2-6 and Comparative Nonaqueous Electrolyte Solutions 2-1 to 2-2.

### <Examples 4-1 to 4-6, Comparative Examples 4-1 to 4-2>

### (Preparation of Nonaqueous Electrolyte Solutions 4-1 to 4-6 and Comparative Nonaqueous Electrolyte Solutions 4-1 to 4-2)

Except that VC was changed to lithium difluorobis(oxalato)phosphate (DFBOP), and the content of Component (I) (or comparative compound) was changed as described in Table 4, Nonaqueous Electrolyte Solutions 4-1 to 4-6 and Comparative Nonaqueous Electrolyte Solutions 4-1 to 4-2 were respectively obtained in the same way as in the preparation of Nonaqueous Electrolyte Solutions 2-1 to 2-6 and Comparative Nonaqueous Electrolyte Solutions 2-1 to 2-2.

### <Examples 5-1 to 5-6, Comparative Examples 5-1 to 5-2>

### (Preparation of Nonaqueous Electrolyte Solutions 5-1 to 5-6 and Comparative Nonaqueous Electrolyte Solutions 5-1 to 5-2)

Except that VC was changed to lithium tetrafluorooxalatophosphate (TFOP), Nonaqueous Electrolyte Solutions 5-1 to 5-6 and Comparative Nonaqueous Electrolyte Solutions 5-1 to 5-2 were respectively obtained in the same way as in the preparation of Nonaqueous Electrolyte Solutions 2-1 to 2-6 and Comparative Nonaqueous Electrolyte Solutions 2-1 to 2-2.

### <Examples 6-1 to 6-6, Comparative Examples 6-1 to 6-2>

### (Preparation of Nonaqueous Electrolyte Solutions 6-1 to 6-6 and Comparative Nonaqueous Electrolyte Solutions 6-1 to 6-2)

Except that VC was changed to lithium bis(fluorosulfonyl)imide (FSI), Nonaqueous Electrolyte Solutions 6-1 to 6-6 and Comparative Nonaqueous Electrolyte Solutions 6-1 to 6-2 were respectively obtained in the same way as in the preparation of Nonaqueous Electrolyte Solutions 2-1 to 2-6 and Comparative Nonaqueous Electrolyte Solutions 2-1 to 2-2.

### <Examples 7-1 to 7-6, Comparative Examples 7-1 to 7-2>

### (Preparation of Nonaqueous Electrolyte Solutions 7-1 to 7-6 and Comparative Nonaqueous Electrolyte Solutions 7-1 to 7-2)

Except that VC was changed to lithium difluorophosphate (DFP), Nonaqueous Electrolyte Solutions 7-1 to 7-6 and Comparative Nonaqueous Electrolyte Solutions 7-1 to 7-2 were respectively obtained in the same way as in the preparation of Nonaqueous Electrolyte Solutions 2-1 to 2-6 and Comparative Nonaqueous Electrolyte Solutions 2-1 to 2-2.

### <Examples 8-1 to 8-6, Comparative Examples 8-1 to 8-2>

### (Preparation of Nonaqueous Electrolyte Solutions 8-1 to 8-6 and Comparative Nonaqueous Electrolyte Solutions 8-1 to 8-2)

Except that VC was changed to lithium fluorosulfonate (FS), Nonaqueous Electrolyte Solutions 8-1 to 8-6 and Comparative Nonaqueous Electrolyte Solutions 8-1 to 8-2 were respectively obtained in the same way as in the preparation of Nonaqueous Electrolyte Solutions 2-1 to 2-6 and Comparative Nonaqueous Electrolyte Solutions 2-1 to 2-2.

### <Example 9-1>

### (Preparation of Nonaqueous Electrolyte Solution 9-1)

EC, FEC, EMC, and DMC were mixed in a volume ratio of EC:FEC:EMC:DMC = 25:2:48:25 in a glove box with a dew point of -60°C or lower (Component (III)). After that, LiPF₆ (Component (II)) was added such that LiPF₆ had a concentration of 1.0 mol/L, with the internal temperature at 40°C or lower kept, Compound (1-1) (Component (I)) corresponding to the compound represented by Formula (1) was added such that Compound (1-1) had a concentration of 1.0% by mass with respect to the total amount of the nonaqueous electrolyte solution, and the mixture was stirred for 1 hour and Compound (1-1) was dissolved to obtain Nonaqueous Electrolyte Solution 9-1 according to Example 9-1.

### <Examples 9-2 to 9-17, Comparative Examples 9-1 to 9-2>

### (Preparation of Nonaqueous Electrolyte Solutions 9-2 to 9-17 and Comparative Nonaqueous Electrolyte Solutions 9-1 to 9-2)

Except that the type and content of Component (I) (or comparative compound) were changed as described in Table 9, Nonaqueous Electrolyte Solutions 9-2 to 9-17 and Comparative Nonaqueous Electrolyte Solutions 9-1 to 9-2 were obtained in the same way as in the preparation of Nonaqueous Electrolyte Solution 9-1.

### <Examples 10-1 to 10-6, Comparative Examples 10-1 to 10-2>

### (Preparation of Nonaqueous Electrolyte Solutions 10-1 to 10-6 and Comparative Nonaqueous Electrolyte Solutions 10-1 to 10-2)

Furthermore, except that VC as another additive was added such that VC had a concentration described in Table 10 and dissolved, Nonaqueous Electrolyte Solutions 10-1 to 10-6 and Comparative Nonaqueous Electrolyte Solutions 10-1 to 10-2 were respectively obtained in the same way as in the preparation of Nonaqueous Electrolyte Solutions 9-5, 9-8, 9-9, 9-11, 9-14, and 9-15, and Comparative Nonaqueous Electrolyte Solutions 9-1 to 9-2.

### <Examples 11-1 to 11-6, Comparative Examples 11-1 to 11-2>

### (Preparation of Nonaqueous Electrolyte Solutions 11-1 to 11-6 and Comparative Nonaqueous Electrolyte Solutions 11-1 to 11-2)

Except that VC was changed to BOB, and the content of Component (I) (or comparative compound) was changed as described in Table 11, Nonaqueous Electrolyte Solutions 11-1 to 11-6 and Comparative Nonaqueous Electrolyte Solutions 11-1 to 11-2 were respectively obtained in the same way as in the preparation of Nonaqueous Electrolyte Solutions 10-1 to 10-6 and Comparative Nonaqueous Electrolyte Solutions 10-1 to 10-2.

### <Examples 12-1 to 12-6, Comparative Examples 12-1 to 12-2>

### (Preparation of Nonaqueous Electrolyte Solutions 12-1 to 12-6 and Comparative Nonaqueous Electrolyte Solutions 12-1 to 12-2)

Except that VC was changed to DFBOP, and the content of Component (I) (or comparative compound) was changed as described in Table 12, Nonaqueous Electrolyte Solutions 12-1 to 12-6 and Comparative Nonaqueous Electrolyte Solutions 12-1 to 12-2 were respectively obtained in the same way as in the preparation of Nonaqueous Electrolyte Solutions 10-1 to 10-6 and Comparative Nonaqueous Electrolyte Solutions 10-1 to 10-2.

### <Examples 13-1 to 13-6, Comparative Examples 13-1 to 13-2>

### (Preparation of Nonaqueous Electrolyte Solutions 13-1 to 13-6 and Comparative Nonaqueous Electrolyte Solutions 13-1 to 13-2)

Except that VC was changed to TFOP, Nonaqueous Electrolyte Solutions 13-1 to 13-6 and Comparative Nonaqueous Electrolyte Solutions 13-1 to 13-2 were respectively obtained in the same way as in the preparation of Nonaqueous Electrolyte Solutions 10-1 to 10-6 and Comparative Nonaqueous Electrolyte Solutions 10-1 to 10-2.

### <Examples 14-1 to 14-6, Comparative Examples 14-1 to 14-2>

### (Preparation of Nonaqueous Electrolyte Solutions 14-1 to 14-6 and Comparative Nonaqueous Electrolyte Solutions 14-1 to 14-2)

Except that VC was changed to FSI, Nonaqueous Electrolyte Solutions 14-1 to 14-6 and Comparative Nonaqueous Electrolyte Solutions 14-1 to 14-2 were respectively obtained in the same way as in the preparation of Nonaqueous Electrolyte Solutions 10-1 to 10-6 and Comparative Nonaqueous Electrolyte Solutions 10-1 to 10-2.

### <Examples 15-1 to 15-6, Comparative Examples 15-1 to 15-2>

### (Preparation of Nonaqueous Electrolyte Solutions 15-1 to 15-6 and Comparative Nonaqueous Electrolyte Solutions 15-1 to 15-2)

Except that VC was changed to DFP, Nonaqueous Electrolyte Solutions 15-1 to 15-6 and Comparative Nonaqueous Electrolyte Solutions 15-1 to 15-2 were respectively obtained in the same way as in the preparation of Nonaqueous Electrolyte Solutions 10-1 to 10-6 and Comparative Nonaqueous Electrolyte Solutions 10-1 to 10-2.

### <Examples 16-1 to 16-6, Comparative Examples 16-1 to 16-2>

### (Preparation of Nonaqueous Electrolyte Solutions 16-1 to 16-6 and Comparative Nonaqueous Electrolyte Solutions 16-1 to 16-2)

Except that VC was changed to FS, Nonaqueous Electrolyte Solutions 16-1 to 16-6 and Comparative Nonaqueous Electrolyte Solutions 16-1 to 16-2 were respectively obtained in the same way as in the preparation of Nonaqueous Electrolyte Solutions 10-1 to 10-6 and Comparative Nonaqueous Electrolyte Solutions 10-1 to 10-2.

### <Example 17-1>

### (Preparation of Nonaqueous Electrolyte Solution 17-1)

PC, EC, FEC, and EMC were mixed in a volume ratio of PC:EC:FEC:EMC = 20:10:2:68 in a glove box with a dew point of -60°C or lower (Component (III)). After that, NaPF₆ (Component (II)) was added such that NaPF₆ had a concentration of 1.0 mol/L, with the internal temperature at 40°C or lower kept, Compound (1-1-Na) (Component (I)) corresponding to the compound represented by Formula (1) was added such that Compound (1-1-Na) had a concentration of 0.5% by mass with respect to the total amount of the nonaqueous electrolyte solution, and the mixture was stirred for 1 hour and Compound (1-1-Na) was dissolved to obtain Nonaqueous Electrolyte Solution 17-1 according to Example 17-1.

### <Examples 17-2 to 17-10, Comparative Examples 17-1 and 17-2>

### (Preparation of Nonaqueous Electrolyte Solutions 17-2 to 17-10 and Comparative Nonaqueous Electrolyte Solutions 17-1 and 17-2)

Except that the type and content of Component (I) (or Comparative Compound (X-Na)) were changed as described in Table 17, Nonaqueous Electrolyte Solutions 17-2 to 17-10 and Comparative Nonaqueous Electrolyte Solutions 17-1 and 17-2 were obtained in the same way as in the preparation of Nonaqueous Electrolyte Solution 17-1.

The structure of Comparative Compound (X-Na) is shown below.

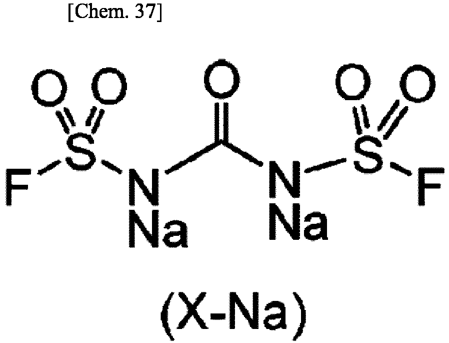

### <Examples 18-1 to 18-5, Comparative Examples 18-1 to 18-2>

### (Preparation of Nonaqueous Electrolyte Solutions 18-1 to 18-5 and Comparative Nonaqueous Electrolyte Solutions 18-1 to 18-2)

Furthermore, except that VC as another additive was added and dissolved such that VC had a concentration described in Table 18, Nonaqueous Electrolyte Solutions 18-1 to 18-5 and Comparative Nonaqueous Electrolyte Solutions 18-1 to 18-2 were respectively obtained in the same way as in the preparation of Nonaqueous Electrolyte Solutions 17-2, 17-4, 17-6, 17-8, and 17-10, and Comparative Nonaqueous Electrolyte Solutions 17-1 to 17-2.

### <Examples 19-1 to 19-5, Comparative Examples 19-1 to 19-2>

### (Preparation of Nonaqueous Electrolyte Solutions 19-1 to 19-5 and Comparative Nonaqueous Electrolyte Solutions 19-1 to 19-2)

Except that VC was changed to 1,3,2-dioxathiolane-2,2-dioxide (DTD) and DTD was added and dissolved such that DTD had a concentration described in Table 19, Nonaqueous Electrolyte Solutions 19-1 to 19-5 and Comparative Nonaqueous Electrolyte Solutions 19-1 to 19-2 were respectively obtained in the same way as in the preparation of Nonaqueous Electrolyte Solutions 18-1 to 18-5 and Comparative Nonaqueous Electrolyte Solutions 18-1 to 18-2.

### <Examples 20-1 to 20-5, Comparative Examples 20-1 to 20-2>

### (Preparation of Nonaqueous Electrolyte Solutions 20-1 to 20-5 and Comparative Nonaqueous Electrolyte Solutions 20-1 to 20-2)

Except that VC was changed to sodium fluorosulfonate (NaSO₃F) and NaSO₃F was added and dissolved such that NaSO₃F had a concentration described in Table 20, Nonaqueous Electrolyte Solutions 20-1 to 20-5 and Comparative Nonaqueous Electrolyte Solutions 20-1 to 20-2 were respectively obtained in the same way as in the preparation of Nonaqueous Electrolyte Solutions 18-1 to 18-5 and Comparative Nonaqueous Electrolyte Solutions 18-1 to 18-2.

### <Examples 21-1 to 21-5, Comparative Examples 21-1 to 21-2>

### (Preparation of Nonaqueous Electrolyte Solutions 21-1 to 21-5 and Comparative Nonaqueous Electrolyte Solutions 21-1 to 21-2)

Except that VC was changed to sodium tetrafluorooxalatophosphate (TFOP-Na) and TFOP-Na was added and dissolved such that TFOP-Na had a concentration described in Table 21, Nonaqueous Electrolyte Solutions 21-1 to 21-5 and Comparative Nonaqueous Electrolyte Solutions 21-1 to 21-2 were respectively obtained in the same way as in the preparation of Nonaqueous Electrolyte Solutions 18-1 to 18-5 and Comparative Nonaqueous Electrolyte Solutions 18-1 and 18-2.

### <Examples 22-1 to 22-5, Comparative Examples 22-1 to 22-2>

### (Preparation of Nonaqueous Electrolyte Solutions 22-1 to 22-5 and Comparative Nonaqueous Electrolyte Solutions 22-1 to 22-2)

Except that VC was changed to sodium difluorophosphate (DFP-Na) and DFP-Na was added and dissolved such that DFP-Na had a concentration described in Table 22, Nonaqueous Electrolyte Solutions 22-1 to 22-5 and Comparative Nonaqueous Electrolyte Solutions 22-1 to 22-2 were respectively obtained in the same way as in the preparation of Nonaqueous Electrolyte Solutions 18-1 to 18-5 and Comparative Nonaqueous Electrolyte Solutions 18-1 to 18-2.

### <Examples 23-1 to 23-5, Comparative Examples 23-1 to 23-2>

### (Preparation of Nonaqueous Electrolyte Solutions 23-1 to 23-5 and Comparative Nonaqueous Electrolyte Solutions 23-1 to 23-2)

Except that VC was changed to sodium difluorooxalatoborate (DFOB-Na) and DFOB-Na was added and dissolved such that DFOB-Na had a concentration described in Table 23, Nonaqueous Electrolyte Solutions 23-1 to 23-5 and Comparative Nonaqueous Electrolyte Solutions 23-1 to 23-2 were respectively obtained in the same way as in the preparation of Nonaqueous Electrolyte Solutions 18-1 to 18-5 and Comparative Nonaqueous Electrolyte Solutions 18-1 and 18-2.

In Tables 1 to 23 below, VC means vinylene carbonate, BOB means lithium bis(oxalato)borate, DFBOP means lithium difluorobis(oxalato)phosphate, TFOP means lithium tetrafluorooxalatophosphate, FSI means lithium bis(fluorosulfonyl)imide, DFP means lithium difluorophosphate, FS means lithium fluorosulfonate, DTD means 1,3,2-dioxathiolane-2,2-dioxide, NaSO₃F means sodium fluorosulfonate, TFOP-Na means sodium tetrafluorooxalatophosphate, DFP-Na means sodium difluorophosphate, and DFOB-Na means sodium difluorooxalatoborate.

In the following Tables 1 to 23, the content of Component (I) (or comparative compound) represents the concentration with respect to the total amount of nonaqueous electrolyte solution. The content of another additive represents the concentration with respect to the total amount of the nonaqueous electrolyte solution.

### [Production of Nonaqueous Electrolyte Solution Battery]

### (Production of Lithium Ion Battery Positive Electrode: NCM622 Positive Electrode)

To 90% by mass of LiNi_{0.6}Co_{0.2}Mn_{0.2}O₂ powder, 5% by mass of polyvinylidene fluoride (hereinafter, also described as PVDF) as a binder and 5% by mass of acetylene black as a conductive material were mixed, and N-methyl-2-pyrrolidone was further added to produce a positive electrode mixture paste. The paste was applied onto both sides of aluminum foil (A1085), and the resultant product was dried and pressed, and then punched into 4 cm × 5 cm to obtain a test NCM622 positive electrode.

### (Production of Lithium Ion Battery Positive Electrode: NCM811 Positive Electrode)

To 92.0% by mass of LiNi_{0.8}Co_{0.1}Mn_{0.1}O₂ powder, 3.5% by mass of PVDF as a binder and 4.5% by mass of acetylene black as a conductive material were mixed, and N-methyl-2-pyrrolidone was further added to produce a positive electrode mixture paste. The paste was applied onto both sides of aluminum foil (A1085), and the resultant product was dried and pressed, and then punched into 4 cm × 5 cm to obtain a test NCM811 positive electrode.

### (Production of Sodium Ion Battery Positive Electrode: NaNi_{0.5}Ti_{0.3}Mn_{0.2}O₂ Positive Electrode)

NaNi_{0.5}Ti_{0.3}Mn_{0.2}O₂ (90% by mass) as a positive electrode active material, acetylene black (5% by mass) as a conductive agent, and PVDF (5% by mass) as a binder were mixed, N-methyl-2-pyrrolidone as a solvent was further added such that N-methyl-2-pyrrolidone is in a content of 50% by mass with respect to a total mass of the positive electrode active material, the conductive agent, and the binder, and a slurry solution was prepared. The slurry solution was applied onto an aluminum foil serving as a positive electrode current collector and dried at 150°C for 12 hours to obtain a test NaNi_{0.5}Ti_{0.3}Mn_{0.2}O₂ positive electrode in which a positive electrode active material layer was formed on the current collector.

### (Production of Natural Graphite Negative Electrode)

Natural graphite powder (92% by mass), conductive material (3% by mass) (HS-100 manufactured by Denka), carbon nanofiber (2% by mass) (VGCF manufactured by Showa Denko), styrene-butadiene rubber (2% by mass), sodium carboxymethylcellulose (1% by mass), and water were mixed, and a slurry solution was prepared. The slurry solution was applied onto a copper foil serving as a negative electrode current collector and dried at 100°C for 12 hours to obtain a test natural graphite negative electrode in which a negative electrode active material layer was formed on the current collector.

### (Production of Silicon-Containing Graphite Negative Electrode)

To 85% by mass of artificial graphite powder, 7% by mass of nanosilicon, 3% by mass of conductive material (HS-100), 2% by mass of carbon nanofiber (VGCF), 2% by mass of styrene-butadiene rubber, 1% by mass of sodium carboxymethylcellulose, and water were mixed, and a slurry solution was prepared. The slurry solution was applied onto a copper foil serving as a negative electrode current collector and dried at 100°C for 12 hours to obtain a test silicon-containing graphite negative electrode in which a negative electrode active material layer was formed on the current collector.

### (Production of Hard Carbon Negative Electrode)

Hard carbon powder (90% by mass) (manufactured by Kureha, Carbotron P) as a negative electrode active material and PVDF (10% by mass) as a binder were mixed, N-methyl pyrrolidone as a solvent was further added such that N-methyl pyrrolidone is in a content of 50% by mass with respect to a total mass of the negative electrode active material and the binder, and a slurry solution was prepared. The slurry solution was applied onto an aluminum foil serving as a negative electrode current collector and dried at 150°C for 12 hours to obtain a test hard carbon negative electrode in which a negative electrode active material layer was formed on the current collector.

### (Production of Nonaqueous Electrolyte Solution Battery)

Under an argon atmosphere at a dew point of -50°C or lower, a terminal was welded to the above NCM622 positive electrode, and both sides of the welded product were then stacked between two polyethylene separators (5 cm × 6 cm), and the outside of the stacked product were stacked between two natural graphite negative electrodes to which a terminal had been welded in advance so that a surface of the negative electrode active material faces a surface of the positive electrode active material. The resultant product was put in an aluminum laminated bag having an opening on one side, the nonaqueous electrolyte solution was vacuum-injected into the bag, and the opening was then sealed with heat to produce aluminum laminated nonaqueous electrolyte solution batteries according to Examples and Comparative Examples. The nonaqueous electrolyte solution used those described in Tables 1 to 8.

In Examples 9-1 to 9-17, Comparative Examples 9-1 to 9-2, Examples 10-1 to 10-6, Comparative Examples 10-1 to 10-2, Examples 11-1 to 11-6, Comparative Examples 11-1 to 11-2, Examples 12-1 to 12-6, Comparative Examples 12-1 to 12-2, Examples 13-1 to 13-6, Comparative Examples 13-1 to 13-2, Examples 14-1 to 14-6, Comparative Examples 14-1 to 14-2, Examples 15-1 to 15-6, Comparative Examples 15-1 to 15-2, Examples 16-1 to 16-6, and Comparative Examples 16-1 to 16-2, NCM811 as the positive electrode and silicon-containing graphite as the negative electrode were used to produce a nonaqueous electrolyte solution battery in the same manner. The nonaqueous electrolyte solution used those described in Tables 9 to 16.

In Examples 17-1 to 17-10, Comparative Examples 17-1 to 17-2, Examples 18-1 to 18-5, Comparative Examples 18-1 to 18-2, Examples 19-1 to 19-5, Comparative Examples 19-1 to 19-2, Examples 20-1 to 20-5, Comparative Examples 20-1 to 20-2, Examples 21-1 to 21-5, Comparative Examples 21-1 to 21-2, Examples 22-1 to 22-5, Comparative Examples 22-1 to 22-2, Examples 23-1 to 23-5, and Comparative Examples 23-1 to 23-2, the NaNi_{0.5}Ti_{0.3}Mn_{0.2}O₂ positive electrode as the positive electrode and the hard carbon negative electrode as the negative electrode were used to produce a nonaqueous electrolyte solution battery (sodium ion battery) in the same manner. The nonaqueous electrolyte solution used those described in Tables 17 to 23.

### [Evaluation] [Lithium Ion Battery: Initial Charge and Discharge]

The produced nonaqueous electrolyte solution battery was put in a 25°C constant temperature bath and, in this state, connected to a charge and discharge device. Charge was performed at 3 mA until 4.3 V. After 4.3 V was maintained for 1 hour, discharge was performed at 6 mA to 2.5 V. This was defined as one charge and discharge cycle, and three cycles in total of charge and discharge were performed to stabilize the battery.

### [Sodium Ion Battery: Initial Charge and Discharge]

The produced nonaqueous electrolyte solution battery was put in a 25°C constant temperature bath and, in this state, connected to a charge and discharge device. Charge was performed at 3 mA until 4.1 V. After 4.1 V was maintained for 1 hour, discharge was performed at 6 mA to 1.5 V. This was defined as one charge and discharge cycle, and three cycles in total of charge and discharge were performed to stabilize the battery.

### [Lithium Ion Battery: Initial Resistance Measurement]

The test cell described above was charged to 4.3 V at 25°C and 6 mA, and then a resistance value was measured by impedance measurement in a -20°C environment.

### [Sodium Ion Battery: Initial Resistance Measurement]

Except that the charge voltage was changed to 4.1 V, an evaluation was performed in the same manner as the lithium ion battery.

### [Lithium Ion Battery: High-Temperature Cycle Characteristics Evaluation]

A charge and discharge test was conducted on the test cell at an environmental temperature of 60°C to evaluate cycle characteristics. The charge and discharge cycle was repeated at a current value of 30 mA by using a constant current constant voltage method with an upper limit charge voltage of 4.3 V and a lower limit discharge voltage of 2.5 V. Further, a degree of deterioration of the cell was evaluated with the discharge capacity retention rate at a 500th cycle in the charge and discharge test at the environmental temperature of 60°C. A "discharge capacity retention rate after a high-temperature cycle" represented by the discharge capacity retention rate at the 500th cycle was obtained by the following formula. A discharge capacity at a first cycle in the charge and discharge test at the environmental temperature of 60°C was defined as an initial discharge capacity.

Discharge capacity retention rate after high-temperature cycle (%) = (discharge capacity at 500th cycle/initial discharge capacity) × 100

### [Sodium Ion Battery: High-Temperature Cycle Characteristics Evaluation]

Except that the upper limit charge voltage was changed to 4.1 V and the lower limit discharge voltage was changed to 1.5 V, an evaluation was performed in the same manner as the lithium ion battery.

### [Lithium Ion Battery: Resistance Measurement After Cycle Test]

The cell after the cycle test was charged to 4.3 V at 25°C and 6 mA, and then a resistance value was measured by impedance measurement in a -20°C environment.

### [Sodium Ion Battery: Resistance Measurement After Cycle Test]

Except that the charge voltage was changed to 4.1 V, an evaluation was performed in the same manner as the lithium ion battery.

In each of Tables 1 to 23, an initial resistance value, the discharge capacity retention rate after the high-temperature cycle, and a value of the resistance after the cycle test of Comparative Example using the comparative nonaqueous electrolyte solution to which neither Component (I) nor a comparative compound was added (Comparative Example 1-1 in Table 1, Comparative Example 2-1 in Table 2, Comparative Example 3-1 in Table 3, Comparative Example 4-1 in Table 4, Comparative Example 5-1 in Table 5, Comparative Example 6-1 in Table 6, Comparative Example 7-1 in Table 7, Comparative Example 8-1 in Table 8, Comparative Example 9-1 in Table 9, Comparative Example 10-1 in Table 10, Comparative Example 11-1 in Table 11, Comparative Example 12-1 in Table 12, Comparative Example 13-1 in Table 13, Comparative Example 14-1 in Table 14, Comparative Example 15-1 in Table 15, Comparative Example 16-1 in Table 16, Comparative Example 17-1 in Table 17, Comparative Example 18-1 in Table 18, Comparative Example 19-1 in Table 19, Comparative Example 20-1 in Table 20, Comparative Example 21-1 in Table 21, Comparative Example 22-1 in Table 22, and Comparative Example 23-1 in Table 23) were respectively defined as 100, and an evaluation result in each of Examples and Comparative Examples was shown as a relative value.

**[Table 1]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 1-1 | Nonaqueous Electrolyte Solution 1-1 | (1-1) | 0.05 | - | - | 98 | 100 | 98 |
| Example 1-2 | Nonaqueous Electrolyte Solution 1-2 | (1-1) | 0.1 | - | - | 90 | 100 | 92 |
| Example 1-3 | Nonaqueous Electrolyte Solution 1-3 | (1-1) | 0.5 | - | - | 61 | 101 | 85 |
| Example 1-4 | Nonaqueous Electrolyte Solution 1-4 | (1-1) | 1.0 | - | - | 58 | 102 | 88 |
| Example 1-5 | Nonaqueous Electrolyte Solution 1-5 | (1-1) | 2.0 | - | - | 74 | 102 | 88 |
| Example 1-6 | Nonaqueous Electrolyte Solution 1-6 | (1-1) | 3.0 | - | - | 89 | 103 | 94 |
| Example 1-7 | Nonaqueous Electrolyte Solution 1-7 | (1-2) | 0.5 | - | - | 52 | 103 | 77 |
| Example 1-8 | Nonaqueous Electrolyte Solution 1-8 | (1-2) | 1.0 | - | - | 50 | 104 | 86 |
| Example 1-9 | Nonaqueous Electrolyte Solution 1-9 | (1-3) | 0.5 | - | - | 53 | 104 | 75 |
| Example 1-10 | Nonaqueous Electrolyte Solution 1-10 | (1-3) | 1.0 | - | - | 49 | 104 | 76 |
| Example 1-11 | Nonaqueous Electrolyte Solution 1-11 | (1-4) | 0.5 | - | - | 50 | 103 | 75 |
| Example 1-12 | Nonaqueous Electrolyte Solution 1-12 | (1-4) | 1.0 | - | - | 47 | 104 | 73 |
| Example 1-13 | Nonaqueous Electrolyte Solution 1-13 | (1-9) | 0.5 | - | - | 82 | 101 | 95 |
| Example 1-14 | Nonaqueous Electrolyte Solution 1-14 | (1-9) | 1.0 | - | - | 83 | 101 | 96 |
| Example 1-15 | Nonaqueous Electrolyte Solution 1-15 | (1-11) | 0.5 | - | - | 55 | 101 | 85 |
| Example 1-16 | Nonaqueous Electrolyte Solution 1-16 | (1-11) | 1.0 | - | - | 52 | 101 | 83 |
| Example 1-17 | Nonaqueous Electrolyte Solution 1-17 | (2-1) | 0.5 | | | 75 | 102 | 86 |
| Example 1-18 | Nonaqueous Electrolyte Solution 1-18 | (3-1) | 0.5 | | | 77 | 103 | 87 |
| Comparative Example 1-1 | Comparative Nonaqueous Electrolyte Solution 1-1 | - | - | - | - | 100 | 100 | 100 |
| Comparative Example 1-2 | Comparative Nonaqueous Electrolyte Solution 1-2 | X | 1.0 | - | - | 77 | 98 | 118 |

**[Table 2]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 2-1 | Nonaqueous Electrolyte Solution 2-1 | (1-1) | 1.0 | VC | 1.0 | 75 | 103 | 83 |
| Example 2-2 | Nonaqueous Electrolyte Solution 2-2 | (1-2) | 1.0 | VC | 1.0 | 68 | 104 | 76 |
| Example 2-3 | Nonaqueous Electrolyte Solution 2-3 | (1-3) | 1.0 | VC | 1.0 | 71 | 104 | 76 |
| Example 2-4 | Nonaqueous Electrolyte Solution 2-4 | (1-4) | 1.0 | VC | 1.0 | 67 | 104 | 73 |
| Example 2-5 | Nonaqueous Electrolyte Solution 2-5 | (1-9) | 1.0 | VC | 1.0 | 85 | 101 | 94 |
| Example 2-6 | Nonaqueous Electrolyte Solution 2-6 | (1-11) | 1.0 | VC | 1.0 | 72 | 101 | 78 |
| Comparative Example 2-1 | Comparative Nonaqueous Electrolyte Solution 2-1 | - | - | VC | 1.0 | 100 | 100 | 100 |
| Comparative Example 2-2 | Comparative Nonaqueous Electrolyte Solution 2-2 | X | 1.0 | VC | 1.0 | 77 | 97 | 109 |

**[Table 3]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 3-1 | Nonaqueous Electrolyte Solution 3-1 | (1-1) | 0.5 | BOB | 1.0 | 71 | 101 | 88 |
| Example 3-2 | Nonaqueous Electrolyte Solution 3-2 | (1-2) | 0.5 | BOB | 1.0 | 40 | 104 | 54 |
| Example 3-3 | Nonaqueous Electrolyte Solution 3-3 | (1-3) | 0.5 | BOB | 1.0 | 44 | 105 | 59 |
| Example 3-4 | Nonaqueous Electrolyte Solution 3-4 | (1-4) | 0.5 | BOB | 1.0 | 50 | 105 | 58 |
| Example 3-5 | Nonaqueous Electrolyte Solution 3-5 | (1-9) | 0.5 | BOB | 1.0 | 85 | 101 | 95 |
| Example 3-6 | Nonaqueous Electrolyte Solution 3-6 | (1-11) | 0.5 | BOB | 1.0 | 44 | 101 | 59 |
| Comparative Example 3-1 | Comparative Nonaqueous Electrolyte Solution 3-1 | - | - | BOB | 1.0 | 100 | 100 | 100 |
| Comparative Example 3-2 | Comparative Nonaqueous Electrolyte Solution 3-2 | X | 0.5 | BOB | 1.0 | 85 | 98 | 111 |

**[Table 4]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 4-1 | Nonaqueous Electrolyte Solution 4-1 | (1-1) | 0.5 | DFBOP | 1.0 | 65 | 101 | 83 |
| Example 4-2 | Nonaqueous Electrolyte Solution 4-2 | (1-2) | 0.5 | DFBOP | 1.0 | 55 | 103 | 70 |
| Example 4-3 | Nonaqueous Electrolyte Solution 4-3 | (1-3) | 0.5 | DFBOP | 1.0 | 43 | 104 | 54 |
| Example 4-4 | Nonaqueous Electrolyte Solution 4-4 | (1-4) | 0.5 | DFBOP | 1.0 | 41 | 104 | 55 |
| Example 4-5 | Nonaqueous Electrolyte Solution 4-5 | (1-9) | 0.5 | DFBOP | 1.0 | 73 | 102 | 92 |
| Example 4-6 | Nonaqueous Electrolyte Solution 4-6 | (1-11) | 0.5 | DFBOP | 1.0 | 58 | 102 | 74 |
| Comparative Example 4-1 | Comparative Nonaqueous Electrolyte Solution 4-1 | - | - | DFBOP | 1.0 | 100 | 100 | 100 |
| Comparative Example 4-2 | Comparative Nonaqueous Electrolyte Solution 4-2 | X | 0.5 | DFBOP | 1.0 | 68 | 97 | 114 |

**[Table 5]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 5-1 | Nonaqueous Electrolyte Solution 5-1 | (1-1) | 1.0 | TFOP | 1.0 | 65 | 103 | 81 |
| Example 5-2 | Nonaqueous Electrolyte Solution 5-2 | (1-2) | 1.0 | TFOP | 1.0 | 54 | 104 | 70 |
| Example 5-3 | Nonaqueous Electrolyte Solution 5-3 | (1-3) | 1.0 | TFOP | 1.0 | 57 | 104 | 75 |
| Example 5-4 | Nonaqueous Electrolyte Solution 5-4 | (1-4) | 1.0 | TFOP | 1.0 | 52 | 105 | 73 |
| Example 5-5 | Nonaqueous Electrolyte Solution 5-5 | (1-9) | 1.0 | TFOP | 1.0 | 80 | 101 | 89 |
| Example 5-6 | Nonaqueous Electrolyte Solution 5-6 | (1-11) | 1.0 | TFOP | 1.0 | 59 | 101 | 75 |
| Comparative Example 5-1 | Comparative Nonaqueous Electrolyte Solution 5-1 | - | - | TFOP | 1.0 | 100 | 100 | 100 |
| Comparative Example 5-2 | Comparative Nonaqueous Electrolyte Solution 5-2 | X | 1.0 | TFOP | 1.0 | 82 | 97 | 107 |

**[Table 6]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 6-1 | Nonaqueous Electrolyte Solution 6-1 | (1-1) | 1.0 | FSI | 1.0 | 78 | 102 | 85 |
| Example 6-2 | Nonaqueous Electrolyte Solution 6-2 | (1-2) | 1.0 | FSI | 1.0 | 70 | 103 | 73 |
| Example 6-3 | Nonaqueous Electrolyte Solution 6-3 | (1-3) | 1.0 | FSI | 1.0 | 71 | 103 | 70 |
| Example 6-4 | Nonaqueous Electrolyte Solution 6-4 | (1-4) | 1.0 | FSI | 1.0 | 69 | 104 | 71 |
| Example 6-5 | Nonaqueous Electrolyte Solution 6-5 | (1-9) | 1.0 | FSI | 1.0 | 82 | 101 | 95 |
| Example 6-6 | Nonaqueous Electrolyte Solution 6-6 | (1-11) | 1.0 | FSI | 1.0 | 74 | 101 | 76 |
| Comparative Example 6-1 | Comparative Nonaqueous Electrolyte Solution 6-1 | - | - | FSI | 1.0 | 100 | 100 | 100 |
| Comparative Example 6-2 | Comparative Nonaqueous Electrolyte Solution 6-2 | X | 1.0 | FSI | 1.0 | 81 | 98 | 108 |

**[Table 7]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 7-1 | Nonaqueous Electrolyte Solution 7-1 | (1-1) | 1.0 | DFP | 1.0 | 79 | 101 | 86 |
| Example 7-2 | Nonaqueous Electrolyte Solution 7-2 | (1-2) | 1.0 | DFP | 1.0 | 69 | 103 | 77 |
| Example 7-3 | Nonaqueous Electrolyte Solution 7-3 | (1-3) | 1.0 | DFP | 1.0 | 73 | 104 | 81 |
| Example 7-4 | Nonaqueous Electrolyte Solution 7-4 | (1-4) | 1.0 | DFP | 1.0 | 73 | 104 | 78 |
| Example 7-5 | Nonaqueous Electrolyte Solution 7-5 | (1-9) | 1.0 | DFP | 1.0 | 88 | 102 | 96 |
| Example 7-6 | Nonaqueous Electrolyte Solution 7-6 | (1-11) | 1.0 | DFP | 1.0 | 73 | 102 | 80 |
| Comparative Example 7-1 | Comparative Nonaqueous Electrolyte Solution 7-1 | - | - | DFP | 1.0 | 100 | 100 | 100 |
| Comparative Example 7-2 | Comparative Nonaqueous Electrolyte Solution 7-2 | X | 1.0 | DFP | 1.0 | 86 | 97 | 115 |

**[Table 8]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 8-1 | Nonaqueous Electrolyte Solution 8-1 | (1-1) | 1.0 | FS | 1.0 | 85 | 103 | 86 |
| Example 8-2 | Nonaqueous Electrolyte Solution 8-2 | (1-2) | 1.0 | FS | 1.0 | 77 | 104 | 73 |
| Example 8-3 | Nonaqueous Electrolyte Solution 8-3 | (1-3) | 1.0 | FS | 1.0 | 83 | 103 | 74 |
| Example 8-4 | Nonaqueous Electrolyte Solution 8-4 | (1-4) | 1.0 | FS | 1.0 | 74 | 104 | 71 |
| Example 8-5 | Nonaqueous Electrolyte Solution 8-5 | (1-9) | 1.0 | FS | 1.0 | 90 | 101 | 94 |
| Example 8-6 | Nonaqueous Electrolyte Solution 8-6 | (1-11) | 1.0 | FS | 1.0 | 81 | 101 | 76 |
| Comparative Example 8-1 | Comparative Nonaqueous Electrolyte Solution 8-1 | - | - | FS | 1.0 | 100 | 100 | 100 |
| Comparative Example 8-2 | Comparative Nonaqueous Electrolyte Solution 8-2 | X | 1.0 | FS | 1.0 | 89 | 97 | 112 |

**[Table 9]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 9-1 | Nonaqueous Electrolyte Solution 9-1 | (1-1) | 1.0 | - | - | 70 | 101 | 74 |
| Example 9-2 | Nonaqueous Electrolyte Solution 9-2 | (1-2) | 0.05 | - | - | 98 | 100 | 99 |
| Example 9-3 | Nonaqueous Electrolyte Solution 9-3 | (1-2) | 0.1 | - | - | 90 | 101 | 95 |
| Example 9-4 | Nonaqueous Electrolyte Solution 9-4 | (1-2) | 0.5 | - | - | 50 | 103 | 81 |
| Example 9-5 | Nonaqueous Electrolyte Solution 9-5 | (1-2) | 1.0 | - | - | 53 | 104 | 83 |
| Example 9-6 | Nonaqueous Electrolyte Solution 9-6 | (1-2) | 2.0 | - | - | 61 | 104 | 88 |
| Example 9-7 | Nonaqueous Electrolyte Solution 9-7 | (1-2) | 3.0 | - | - | 70 | 104 | 90 |
| Example 9-8 | Nonaqueous Electrolyte Solution 9-8 | (1-3) | 1.0 | - | - | 66 | 103 | 75 |
| Example 9-9 | Nonaqueous Electrolyte Solution 9-9 | (1-4) | 1.0 | - | - | 55 | 104 | 77 |
| Example 9-10 | Nonaqueous Electrolyte Solution 9-10 | (1-5) | 1.0 | - | - | 57 | 102 | 73 |
| Example 9-11 | Nonaqueous Electrolyte Solution 9-11 | (1-6) | 1.0 | - | - | 62 | 101 | 81 |
| Example 9-12 | Nonaqueous Electrolyte Solution 9-12 | (1-7) | 1.0 | - | - | 68 | 102 | 82 |
| Example 9-13 | Nonaqueous Electrolyte Solution 9-13 | (1-8) | 1.0 | - | - | 72 | 104 | 80 |
| Example 9-14 | Nonaqueous Electrolyte Solution 9-14 | (1-9) | 1.0 | - | - | 90 | 101 | 96 |
| Example 9-15 | Nonaqueous Electrolyte Solution 9-15 | (1-11) | 1.0 | - | - | 58 | 101 | 88 |
| Example 9-16 | Nonaqueous Electrolyte Solution 9-16 | (2-1) | 0.5 | - | - | 72 | 104 | 80 |
| Example 9-17 | Nonaqueous Electrolyte Solution 9-17 | (3-1) | 0.5 | - | - | 73 | 104 | 82 |
| Comparative Example 9-1 | Comparative Nonaqueous Electrolyte Solution 9-1 | - | - | - | - | 100 | 100 | 100 |
| Comparative Example 9-2 | Comparative Nonaqueous Electrolyte Solution 9-2 | X | 1.0 | - | - | 78 | 96 | 123 |

**[Table 10]**

| | Nonaqueous electrolyte solution | Component (I) or | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 10-1 | Nonaqueous Electrolyte Solution 10-1 | (1-2) | 1.0 | VC | 1.0 | 63 | 105 | 68 |
| Example 10-2 | Nonaqueous Electrolyte Solution 10-2 | (1-3) | 1.0 | VC | 1.0 | 67 | 105 | 71 |
| Example 10-3 | Nonaqueous Electrolyte Solution 10-3 | (1-4) | 1.0 | VC | 1.0 | 64 | 104 | 69 |
| Example 10-4 | Nonaqueous Electrolyte Solution 10-4 | (1-6) | 1.0 | VC | 1.0 | 71 | 103 | 77 |
| Example 10-5 | Nonaqueous Electrolyte Solution 10-5 | (1-9) | 1.0 | VC | 1.0 | 90 | 103 | 93 |
| Example 10-6 | Nonaqueous Electrolyte Solution 10-6 | (1-11) | 1.0 | VC | 1.0 | 68 | 101 | 73 |
| Comparative Example 10-1 | Comparative Nonaqueous Electrolyte Solution 10-1 | - | - | VC | 1.0 | 100 | 100 | 100 |
| Comparative Example 10-2 | Comparative Nonaqueous Electrolyte Solution 10-2 | X | 1.0 | VC | 1.0 | 81 | 97 | 113 |

**[Table 11]**

| | Nonaqueous electrolyte solution | Component (I) or | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 11-1 | Nonaqueous Electrolyte Solution 11-1 | (1-2) | 0.5 | BOB | 1.0 | 48 | 104 | 66 |
| Example 11-2 | Nonaqueous Electrolyte Solution 11-2 | (1-3) | 0.5 | BOB | 1.0 | 57 | 104 | 75 |
| Example 11-3 | Nonaqueous Electrolyte Solution 11-3 | (1-4) | 0.5 | BOB | 1.0 | 55 | 103 | 70 |
| Example 11-4 | Nonaqueous Electrolyte Solution 11-4 | (1-6) | 0.5 | BOB | 1.0 | 65 | 102 | 74 |
| Example 11-5 | Nonaqueous Electrolyte Solution 11-5 | (1-9) | 0.5 | BOB | 1.0 | 88 | 101 | 97 |
| Example 11-6 | Nonaqueous Electrolyte Solution 11-6 | (1-11) | 0.5 | BOB | 1.0 | 61 | 101 | 79 |
| Comparative Example 11-1 | Comparative Nonaqueous Electrolyte Solution 11-1 | - | - | BOB | 1.0 | 100 | 100 | 100 |
| Comparative Example 11-2 | Comparative Nonaqueous Electrolyte Solution 11-2 | X | 0.5 | BOB | 1.0 | 88 | 96 | 117 |

**[Table 12]**

| | Nonaqueous electrolyte solution | Component (I) or | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 12-1 | Nonaqueous Electrolyte Solution 12-1 | (1-2) | 0.5 | DFBOP | 1.0 | 49 | 103 | 71 |
| Example 12-2 | Nonaqueous Electrolyte Solution 12-2 | (1-3) | 0.5 | DFBOP | 1.0 | 48 | 103 | 69 |
| Example 12-3 | Nonaqueous Electrolyte Solution 12-3 | (1-4) | 0.5 | DFBOP | 1.0 | 52 | 104 | 73 |
| Example 12-4 | Nonaqueous Electrolyte Solution 12-4 | (1-6) | 0.5 | DFBOP | 1.0 | 62 | 104 | 77 |
| Example 12-5 | Nonaqueous Electrolyte Solution 12-5 | (1-9) | 0.5 | DFBOP | 1.0 | 91 | 101 | 96 |
| Example 12-6 | Nonaqueous Electrolyte Solution 12-6 | (1-11) | 0.5 | DFBOP | 1.0 | 53 | 101 | 75 |
| Comparative Example 12-1 | Comparative Nonaqueous Electrolyte Solution 12-1 | - | - | DFBOP | 1.0 | 100 | 100 | 100 |
| Comparative Example 12-2 | Comparative Nonaqueous Electrolyte Solution 12-2 | X | 0.5 | DFBOP | 1.0 | 72 | 95 | 112 |

**[Table 13]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 13-1 | Nonaqueous Electrolyte Solution 13-1 | (1-2) | 1.0 | TFOP | 1.0 | 60 | 103 | 80 |
| Example 13-2 | Nonaqueous Electrolyte Solution 13-2 | (1-3) | 1.0 | TFOP | 1.0 | 58 | 104 | 72 |
| Example 13-3 | Nonaqueous Electrolyte Solution 13-3 | (1-4) | 1.0 | TFOP | 1.0 | 57 | 104 | 79 |
| Example 13-4 | Nonaqueous Electrolyte Solution 13-4 | (1-6) | 1.0 | TFOP | 1.0 | 73 | 103 | 85 |
| Example 13-5 | Nonaqueous Electrolyte Solution 13-5 | (1-9) | 1.0 | TFOP | 1.0 | 86 | 101 | 95 |
| Example 13-6 | Nonaqueous Electrolyte Solution 13-6 | (1-11) | 1.0 | TFOP | 1.0 | 64 | 101 | 84 |
| Comparative Example 13-1 | Comparative Nonaqueous Electrolyte Solution 13-1 | - | - | TFOP | 1.0 | 100 | 100 | 100 |
| Comparative Example 13-2 | Comparative Nonaqueous Electrolyte Solution 13-2 | X | 1.0 | TFOP | 1.0 | 84 | 99 | 112 |

**[Table 14]**

| | Nonaqueous electrolyte solution | Component (I) or | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 14-1 | Nonaqueous Electrolyte Solution 14-1 | (1-2) | 1.0 | FSI | 1.0 | 61 | 103 | 73 |
| Example 14-2 | Nonaqueous Electrolyte Solution 14-2 | (1-3) | 1.0 | FSI | 1.0 | 59 | 103 | 79 |
| Example 14-3 | Nonaqueous Electrolyte Solution 14-3 | (1-4) | 1.0 | FSI | 1.0 | 63 | 102 | 76 |
| Example 14-4 | Nonaqueous Electrolyte Solution 14-4 | (1-6) | 1.0 | FSI | 1.0 | 72 | 102 | 83 |
| Example 14-5 | Nonaqueous Electrolyte Solution 14-5 | (1-9) | 1.0 | FSI | 1.0 | 87 | 101 | 95 |
| Example 14-6 | Nonaqueous Electrolyte Solution 14-6 | (1-11) | 1.0 | FSI | 1.0 | 65 | 101 | 78 |
| Comparative Example 14-1 | Comparative Nonaqueous Electrolyte Solution 14-1 | - | - | FSI | 1.0 | 100 | 100 | 100 |
| Comparative Example 14-2 | Comparative Nonaqueous Electrolyte Solution 14-2 | X | 1.0 | FSI | 1.0 | 83 | 98 | 113 |

**[Table 15]**

| | Nonaqueous electrolyte solution | Component (I) or | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 15-1 | Nonaqueous Electrolyte Solution 15-1 | (1-2) | 1.0 | DFP | 1.0 | 72 | 103 | 76 |
| Example 15-2 | Nonaqueous Electrolyte Solution 15-2 | (1-3) | 1.0 | DFP | 1.0 | 70 | 103 | 75 |
| Example 15-3 | Nonaqueous Electrolyte Solution 15-3 | (1-4) | 1.0 | DFP | 1.0 | 71 | 104 | 73 |
| Example 15-4 | Nonaqueous Electrolyte Solution 15-4 | (1-6) | 1.0 | DFP | 1.0 | 74 | 104 | 80 |
| Example 15-5 | Nonaqueous Electrolyte Solution 15-5 | (1-9) | 1.0 | DFP | 1.0 | 89 | 101 | 94 |
| Example 15-6 | Nonaqueous Electrolyte Solution 15-6 | (1-11) | 1.0 | DFP | 1.0 | 76 | 101 | 80 |
| Comparative Example 15-1 | Comparative Nonaqueous Electrolyte Solution 15-1 | - | - | DFP | 1.0 | 100 | 100 | 100 |
| Comparative Example 15-2 | Comparative Nonaqueous Electrolyte Solution 15-2 | X | 1.0 | DFP | 1.0 | 88 | 99 | 116 |

**[Table 16]**

| | Nonaqueous electrolyte solution | Component (I) or | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 16-1 | Nonaqueous Electrolyte Solution 16-1 | (1-2) | 1.0 | FS | 1.0 | 76 | 102 | 72 |
| Example 16-2 | Nonaqueous Electrolyte Solution 16-2 | (1-3) | 1.0 | FS | 1.0 | 75 | 102 | 74 |
| Example 16-3 | Nonaqueous Electrolyte Solution 16-3 | (1-4) | 1.0 | FS | 1.0 | 76 | 103 | 74 |
| Example 16-4 | Nonaqueous Electrolyte Solution 16-4 | (1-6) | 1.0 | FS | 1.0 | 80 | 104 | 86 |
| Example 16-5 | Nonaqueous Electrolyte Solution 16-5 | (1-9) | 1.0 | FS | 1.0 | 94 | 101 | 96 |
| Example 16-6 | Nonaqueous Electrolyte Solution 16-6 | (1-11) | 1.0 | FS | 1.0 | 80 | 101 | 76 |
| Comparative Example 16-1 | Comparative Nonaqueous Electrolyte Solution 16-1 | - | - | FS | 1.0 | 100 | 100 | 100 |
| Comparative Example 16-2 | Comparative Nonaqueous Electrolyte Solution 16-2 | X | 1.0 | FS | 1.0 | 85 | 98 | 119 |

**[Table 17]**

| | Nonaqueous electrolyte solution | Component (I) or comparative compound | | Another additive | | Initial resistanc e (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 17-1 | Nonaqueous Electrolyte Solution 17-1 | (1-1-Na) | 0.5 | - | - | 87 | 101 | 80 |
| Example 17-2 | Nonaqueous Electrolyte Solution 17-2 | (1-1-Na) | 1.0 | - | - | 86 | 104 | 80 |
| Example 17-3 | Nonaqueous Electrolyte Solution 17-3 | (1-2-Na) | 0.5 | - | - | 78 | 102 | 78 |
| Example 17-4 | Nonaqueous Electrolyte Solution 17-4 | (1-2-Na) | 1.0 | - | - | 73 | 103 | 76 |
| Example 17-5 | Nonaqueous Electrolyte Solution 17-5 | (1-3-Na) | 0.5 | - | - | 74 | 102 | 77 |
| Example 17-6 | Nonaqueous Electrolyte Solution 17-6 | (1-3-Na) | 1.0 | - | - | 71 | 104 | 78 |
| Example 17-7 | Nonaqueous Electrolyte Solution 17-7 | (1-4-Na) | 0.5 | - | - | 74 | 104 | 75 |
| Example 17-8 | Nonaqueous Electrolyte Solution 17-8 | (1-4-Na) | 1.0 | - | - | 69 | 106 | 72 |
| Example 17-9 | Nonaqueous Electrolyte Solution 17-9 | (1-11-Na) | 0.5 | - | - | 82 | 102 | 83 |
| Example 17-10 | Nonaqueous Electrolyte Solution 17-10 | (1-11-Na) | 1.0 | - | - | 77 | 103 | 80 |
| Comparative Example 17-1 | Comparative Nonaqueous Electrolyte Solution 17-1 | - | - | - | - | 100 | 100 | 100 |
| Comparative Example 17-2 | Comparative Nonaqueous Electrolyte Solution 17-2 | X-Na | 1.0 | - | - | 88 | 99 | 119 |

**[Table 18]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 18-1 | Nonaqueous Electrolyte Solution 18-1 | (1-1-Na) | 1.0 | VC | 1.0 | 80 | 102 | 85 |
| Example 18-2 | Nonaqueous Electrolyte Solution 18-2 | (1-2-Na) | 1.0 | VC | 1.0 | 76 | 104 | 80 |
| Example 18-3 | Nonaqueous Electrolyte Solution 18-3 | (1-3-Na) | 1.0 | VC | 1.0 | 77 | 105 | 79 |
| Example 18-4 | Nonaqueous Electrolyte Solution 18-4 | (1-4-Na) | 1.0 | VC | 1.0 | 71 | 104 | 77 |
| Example 18-5 | Nonaqueous Electrolyte Solution 18-5 | (1-11-Na) | 1.0 | VC | 1.0 | 80 | 103 | 83 |
| Comparative Example 18-1 | Comparative Nonaqueous Electrolyte Solution 18-1 | - | - | VC | 1.0 | 100 | 100 | 100 |
| Comparative Example 18-2 | Comparative Nonaqueous Electrolyte Solution 18-2 | X-Na | 1.0 | VC | 1.0 | 83 | 101 | 106 |

**[Table 19]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 19-1 | Comparative Nonaqueous Electrolyte Solution 19-1 | (1-1-Na) | 1.0 | DTD | 1.0 | 82 | 104 | 88 |
| Example 19-2 | Comparative Nonaqueous Electrolyte Solution 19-2 | (1-2-Na) | 1.0 | DTD | 1.0 | 78 | 104 | 75 |
| Example 19-3 | Comparative Nonaqueous Electrolyte Solution 19-3 | (1-3-Na) | 1.0 | DTD | 1.0 | 75 | 103 | 72 |
| Example 19-4 | Comparative Nonaqueous Electrolyte Solution 19-4 | (1-4-Na) | 1.0 | DTD | 1.0 | 71 | 106 | 74 |
| Example 19-5 | Comparative Nonaqueous Electrolyte Solution 19-5 | (1-11-Na) | 1.0 | DTD | 1.0 | 80 | 104 | 78 |
| Comparative Example 19-1 | Comparative Nonaqueous Electrolyte Solution 19-1 | - | - | DTD | 1.0 | 100 | 100 | 100 |
| Comparative Example 19-2 | Comparative Nonaqueous Electrolyte Solution 19-2 | X-Na | 1.0 | DTD | 1.0 | 85 | 101 | 105 |

**[Table 20]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 20-1 | Nonaqueous Electrolyte Solution 20-1 | (1-1-Na) | 1.0 | NaSOsF | 1.0 | 85 | 103 | 90 |
| Example 20-2 | Nonaqueous Electrolyte Solution 20-2 | (1-2-Na) | 1.0 | NaSO₃F | 1.0 | 84 | 105 | 85 |
| Example 20-3 | Nonaqueous Electrolyte Solution 20-3 | (1-3-Na) | 1.0 | NaSOsF | 1.0 | 80 | 106 | 85 |
| Example 20-4 | Nonaqueous Electrolyte Solution 20-4 | (1-4-Na) | 1.0 | NaSO₃F | 1.0 | 77 | 106 | 81 |
| Example 20-5 | Nonaqueous Electrolyte Solution 20-5 | (1-11-Na) | 1.0 | NaSO₃F | 1.0 | 87 | 105 | 88 |
| Comparative Example 20-1 | Comparative Nonaqueous Electrolyte Solution 20-1 | - | - | NaSOsF | 1.0 | 100 | 100 | 100 |
| Comparative Example 20-2 | Comparative Nonaqueous Electrolyte Solution 20-2 | X-Na | 1.0 | NaSOsF | 1.0 | 90 | 100 | 108 |

**[Table 21]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 21-1 | Nonaqueous Electrolyte Solution 21-1 | (1-1-Na) | 1.0 | TFOP-Na | 1.0 | 80 | 103 | 88 |
| Example 21-2 | Nonaqueous Electrolyte Solution 21-2 | (1-2-Na) | 1.0 | TFOP-Na | 1.0 | 81 | 104 | 78 |
| Example 21-3 | Nonaqueous Electrolyte Solution 21-3 | (1-3-Na) | 1.0 | TFOP-Na | 1.0 | 77 | 105 | 79 |
| Example 21-4 | Nonaqueous Electrolyte Solution 21-4 | (1-4-Na) | 1.0 | TFOP-Na | 1.0 | 72 | 104 | 77 |
| Example 21-5 | Nonaqueous Electrolyte Solution 21-5 | (1-11-Na) | 1.0 | TFOP-Na | 1.0 | 83 | 104 | 80 |
| Comparative Example 21-1 | Comparative Nonaqueous Electrolyte Solution 20-1 | - | - | TFOP-Na | 1.0 | 100 | 100 | 100 |
| Comparative Example 21-2 | Comparative Nonaqueous Electrolyte Solution 20-2 | X-Na | 1.0 | TFOP-Na | 1.0 | 84 | 101 | 105 |

**[Table 22]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 22-1 | Nonaqueous Electrolyte Solution 22-1 | (1-1-Na) | 1.0 | DFP-Na | 1.0 | 84 | 103 | 88 |
| Example 22-2 | Nonaqueous Electrolyte Solution 22-2 | (1-2-Na) | 1.0 | DFP-Na | 1.0 | 81 | 105 | 86 |
| Example 22-3 | Nonaqueous Electrolyte Solution 22-3 | (1-3-Na) | 1.0 | DFP-Na | 1.0 | 78 | 107 | 79 |
| Example 22-4 | Nonaqueous Electrolyte Solution 22-4 | (1-4-Na) | 1.0 | DFP-Na | 1.0 | 76 | 107 | 80 |
| Example 22-5 | Nonaqueous Electrolyte Solution 22-5 | (1-11-Na) | 1.0 | DFP-Na | 1.0 | 82 | 104 | 86 |
| Comparative Example 22-1 | Comparative Nonaqueous Electrolyte Solution 22-1 | - | - | DFP-Na | 1.0 | 100 | 100 | 100 |
| Comparative Example 22-2 | Comparative Nonaqueous Electrolyte Solution 22-2 | X-Na | 1.0 | DFP-Na | 1.0 | 89 | 101 | 109 |

**[Table 23]**

| | Nonaqueous electrolyte solution | Component (I) or comparative compound | | Another additive | | Initial resistance (relative value) | Discharge capacity retention rate (relative value) | Resistance after cycle test (relative value) |
|---|---|---|---|---|---|---|---|---|
| | | Type | Content [% by mass] | Type | Content [% by mass] | | | |
| Example 23-1 | Nonaqueous Electrolyte Solution 23-1 | (1-1-Na) | 1.0 | DFOB-Na | 1.0 | 81 | 104 | 84 |
| Example 23-2 | Nonaqueous Electrolyte Solution 23-2 | (1-2-Na) | 1.0 | DFOB-Na | 1.0 | 77 | 104 | 80 |
| Example 23-3 | Nonaqueous Electrolyte Solution 23-3 | (1-3-Na) | 1.0 | DFOB-Na | 1.0 | 76 | 106 | 74 |
| Example 23-4 | Nonaqueous Electrolyte Solution 23-4 | (1-4-Na) | 1.0 | DFOB-Na | 1.0 | 72 | 107 | 73 |
| Example 23-5 | Nonaqueous Electrolyte Solution 23-5 | (1-11-Na) | 1.0 | DFOB-Na | 1.0 | 79 | 104 | 83 |
| Comparative Example 23-1 | Comparative Nonaqueous Electrolyte Solution 23-1 | - | - | DFOB-Na | 1.0 | 100 | 100 | 100 |
| Comparative Example 23-2 | Comparative Nonaqueous Electrolyte Solution 23-2 | X-Na | 1.0 | DFOB-Na | 1.0 | 85 | 99 | 107 |

As is clear from Tables 1 to 23, it was found that the nonaqueous electrolyte solution batteries using the nonaqueous electrolyte solutions containing Component (I) of the present disclosure had low initial resistance and could suppress an increase in resistance during the high-temperature cycle. It could be seen that the nonaqueous electrolyte solutions containing Component (I) achieved high-temperature cycle characteristics equal to or higher than those of the nonaqueous electrolyte solutions according to Comparative Examples that do not contain Component (I) of the present disclosure.

### INDUSTRIAL APPLICABILITY

The present disclosure allows for providing a nonaqueous electrolyte solution and a nonaqueous electrolyte solution battery that can reduce an initial resistance value and suppress an increase during a high-temperature cycle. The present disclosure also allows for providing a compound that can be suitably used in the above nonaqueous electrolyte solution.

Although the present disclosure has been described in detail and with reference to specific examples, it will be apparent to those skilled in the art that various changes and modifications can be made without departing from the spirit and scope of the present disclosure.

The present application is based on a Japanese Patent Application No. 2021-167757 filed on October 12, 2021, the contents of which are incorporated herein by reference.

## Claims

1. A nonaqueous electrolyte solution, comprising:
(I) at least one selected from the group consisting of a compound represented by the following Formula (1), a compound represented by the following Formula (2), and a compound represented by the following Formula (3), 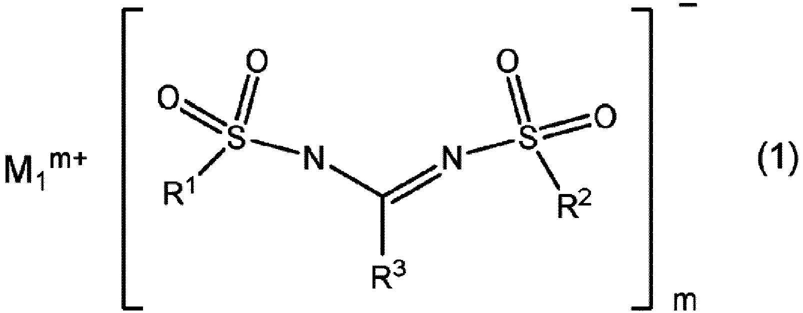
where, M₁^{m+} represents a proton, a metal cation, or an onium cation, and m represents a valence of the cation,
R¹ and R² each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more),
R³ represents a halogen atom, a perfluoroalkyl group having 1 to 4 carbon atoms, a perfluoroalkenyl group having 2 to 4 carbon atoms, a perfluoroalkynyl group having 2 to 4 carbon atoms, a cyano group, an amino group, an aminocarbonyl group, a perfluoroalkylcarbonyl group having 2 to 4 carbon atoms, a hydrocarbon group having 1 to 10 carbon atoms, the hydrocarbon group may have a hetero atom or a halogen atom and may have a branched chain or a cyclic structure if the number of carbon atoms is 3 or more, -C(=O)-R', where R' represents a hydrocarbon group having 1 to 10 carbon atoms, the hydrocarbon group may have a hetero atom or a halogen atom and may have a branched chain or cyclic structure if the number of carbon atoms is 3 or more, an alkoxy group having 1 to 10 carbon atoms, an alkenyloxy group having 2 to 10 carbon atoms, an alkynyloxy group having 2 to 10 carbon atoms, where the alkoxy group, alkenyloxy group, and alkynyloxy group described above may further have a hetero atom or a halogen atom and may have a branched chain or cyclic structure if the number of carbon atoms is 3 or more, an aryloxy group having 6 to 10 carbon atoms, or -O-(Mₐ^{p+})_{q}, where Mₑ^{p+} represents a proton, a metal cation, or an onium cation, p represents a valence of the cation and q represents a number to satisfy p × q = 1, -R^{a}-S(=O)₂(R^{b}), or -R^{c}-P(=O)(R^{d})(R^{e}).
R^{a} and R^{c} each independently represent a perfluoroalkylene group having 1 to 4 carbon atoms or a hydrocarbon group having 1 to 10 carbon atoms, where the hydrocarbon group may have a hetero atom or a halogen atom and may have a branched chain or cyclic structure if the number of carbon atoms is 3 or more,
R^{b} represents a halogen atom, a perfluoroalkyl group having 1 to 4 carbon atoms, a hydrocarbon group having 1 to 10 carbon atoms, where the hydrocarbon group may have a hetero atom or a halogen atom and may have a branched chain or cyclic structure if the number of carbon atoms is 3 or more, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₂^{r+})ₛ, where M₂^{r+} represents a proton, a metal cation, or an onium cation, r represents a valence of the cation, and s represents a number to satisfy r × s = 1,
R^{d} and R^{e} each independently represent a halogen atom, a perfluoroalkyl group having 1 to 4 carbon atoms, a hydrocarbon group having 1 to 10 carbon atoms, where the hydrocarbon group may have a hetero atom or a halogen atom and may have a branched chain or cyclic structure if the number of carbon atoms is 3 or more, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₃^{t+})ᵤ, where M₃²⁺ represents a proton, a metal cation, or an onium cation, t represents a valence of the cation and u represents a number to satisfy t × u = 1, 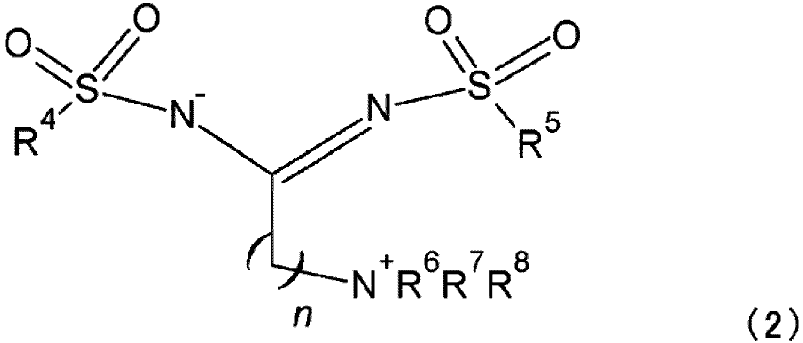
where R⁴ and R⁵ each independently represent a halogen atom, a perfluoroalkyl group having 1 to 4 carbon atoms, or a hydrocarbon group having 1 to 10 carbon atoms, where the hydrocarbon group may have a hetero atom or a halogen atom and may have a branched chain or a cyclic structure if the number of carbon atoms is 3 or more,
R⁶ to R⁸ each independently represent a hydrogen atom or a hydrocarbon group having 1 to 10 carbon atoms, where the hydrocarbon group may have a hetero atom or a halogen atom and may have a branched chain or cyclic structure if the number of carbon atoms is 3 or more,
n represents an integer of 1 to 4, and 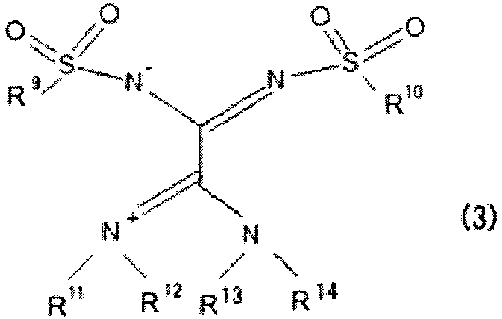
where, R⁹ and R¹⁰ each independently represent a halogen atom, a perfluoroalkyl group having 1 to 4 carbon atoms, or a hydrocarbon group having 1 to 10 carbon atoms, where the hydrocarbon group may have a hetero atom or a halogen atom, and may have a branched chain or a cyclic structure if the number of carbon atoms is 3 or more,
R¹¹ to R¹⁴ each independently represent a hydrogen atom or a hydrocarbon group having 1 to 10 carbon atoms, where the hydrocarbon group may have a hetero atom or a halogen atom and may have a branched chain or cyclic structure if the number of carbon atoms is 3 or more, R¹² and R¹³ may bond to each other to form a ring.

2. The nonaqueous electrolyte solution according to claim 1,
wherein at least one of R¹ and R² in the Formula (1) represents a fluorine atom.

3. The nonaqueous electrolyte solution according to claim 1,
wherein at least one of R⁴ and R⁵ in the Formula (2) represents a fluorine atom.

4. The nonaqueous electrolyte solution according to claim 1,
wherein at least one of R⁹ and R¹⁰ in the Formula (3) represents a fluorine atom.

5. The nonaqueous electrolyte solution according to any one of claims 1 to 4, further comprising:
(II) a solute.

6. The nonaqueous electrolyte solution according to any one of claims 1 to 4, further comprising:
(III) a nonaqueous organic solvent.

7. The nonaqueous electrolyte solution according to claim 5,
wherein the (II) solute is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

8. The nonaqueous electrolyte solution according to claim 6,
wherein the (III) nonaqueous organic solvent comprises at least one selected from the group consisting of a cyclic ester, a chain ester, a cyclic ether, a chain ether, a sulfone compound, a sulfoxide compound, and an ionic liquid.

9. The nonaqueous electrolyte solution according to claim 8,
wherein the nonaqueous organic solvent comprises a cyclic ester, and the cyclic ester comprises cyclic carbonate.

10. The nonaqueous electrolyte solution according to claim 9,
wherein the cyclic carbonate comprises at least one selected from the group consisting of ethylene carbonate, propylene carbonate, and fluoroethylene carbonate.

11. The nonaqueous electrolyte solution according to claim 8,
wherein the nonaqueous organic solvent comprises a chain ester, and the chain ester comprises chain carbonate.

12. The nonaqueous electrolyte solution according to claim 11, wherein
the chain carbonate comprises at least one selected from the group consisting of ethyl methyl carbonate, dimethyl carbonate, diethyl carbonate, and methyl propyl carbonate.

13. The nonaqueous electrolyte solution according to any one of claims 1 to 4,
wherein a content of the (I) is 0.01% by mass to 5.0% by mass with respect to the total amount of the nonaqueous electrolyte solution.

14. The nonaqueous electrolyte solution according to any one of claims 1 to 4, further comprising:
at least one selected from vinylene carbonate, bis(oxalato)borate, difluorooxalatoborate, difluorobis(oxalato)phosphate, tetrafluorooxalatophosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene.

15. A nonaqueous electrolyte solution battery, at least comprising:
a positive electrode;
a negative electrode;
a separator; and
the nonaqueous electrolyte solution according to any one of claims 1 to 4.

16. A compound represented by the following Formula (1), a compound represented by the following Formula (2), or a compound represented by the following Formula (3), 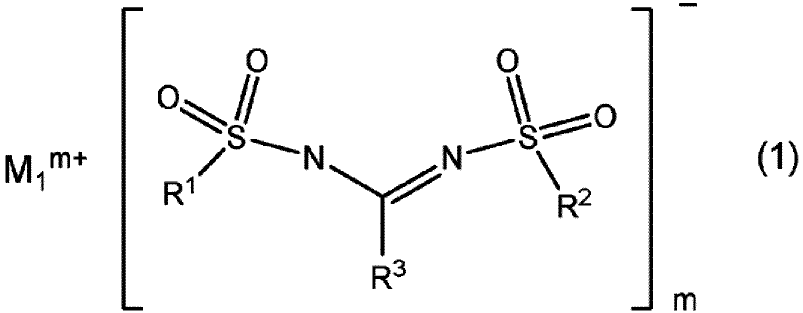
where, M₁^{m+} represents a proton, a metal cation, or an onium cation, and m represents a valence of the cation,
R¹ and R² each independently represent a halogen atom, a perfluoroalkyl group having 1 to 4 carbon atoms, or a hydrocarbon group having 1 to 10 carbon atoms (the hydrocarbon group may have a hetero atom or a halogen atom, and may have a branched chain or a cyclic structure if the number of carbon atoms is 3 or more),
R³ represents a halogen atom, a perfluoroalkyl group having 1 to 4 carbon atoms, a perfluoroalkenyl group having 2 to 4 carbon atoms, a perfluoroalkynyl group having 2 to 4 carbon atoms, a cyano group, an amino group, an aminocarbonyl group, a perfluoroalkylcarbonyl group having 2 to 4 carbon atoms, a hydrocarbon group having 1 to 10 carbon atoms, the hydrocarbon group may have a hetero atom or a halogen atom and may have a branched chain or a cyclic structure if the number of carbon atoms is 3 or more, -C(=O)-R', where R' represents a hydrocarbon group having 1 to 10 carbon atoms, the hydrocarbon group may have a hetero atom or a halogen atom and may have a branched chain or cyclic structure if the number of carbon atoms is 3 or more, an alkoxy group having 1 to 10 carbon atoms, an alkenyloxy group having 2 to 10 carbon atoms, an alkynyloxy group having 2 to 10 carbon atoms, where the alkoxy group, alkenyloxy group, and alkynyloxy group described above may further have a hetero atom or a halogen atom and may have a branched chain or cyclic structure if the number of carbon atoms is 3 or more, an aryloxy group having 6 to 10 carbon atoms, or -O-(Mₐ^{p+})_{q}, where Mₐ^{p+} represents a proton, a metal cation, or an onium cation, p represents a valence of the cation and q represents a number to satisfy p × q = 1, -R^{a}-S(=O)₂(R^{b}), or -R^{c}-P(=O)(R^{d})(R^{e}).
R^{a} and R^{c} each independently represent a perfluoroalkylene group having 1 to 4 carbon atoms or a hydrocarbon group having 1 to 10 carbon atoms, where the hydrocarbon group may have a hetero atom or a halogen atom and may have a branched chain or cyclic structure if the number of carbon atoms is 3 or more,
R^{b} represents a halogen atom, a perfluoroalkyl group having 1 to 4 carbon atoms, a hydrocarbon group having 1 to 10 carbon atoms, where the hydrocarbon group may have a hetero atom or a halogen atom and may have a branched chain or cyclic structure if the number of carbon atoms is 3 or more, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₂^{r+})ₛ, where M₂^{r+} represents a proton, a metal cation, or an onium cation, r represents a valence of the cation, and s represents a number to satisfy r × s = 1,
R^{d} and R^{e} each independently represent a halogen atom, a perfluoroalkyl group having 1 to 4 carbon atoms, a hydrocarbon group having 1 to 10 carbon atoms, where the hydrocarbon group may have a hetero atom or a halogen atom and may have a branched chain or cyclic structure if the number of carbon atoms is 3 or more, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, an aryloxy group having 6 to 10 carbon atoms, or -O⁻(M₃^{t+})ᵤ, where M₃^{t+} represents a proton, a metal cation, or an onium cation, t represents a valence of the cation and u represents a number to satisfy t × u = 1, 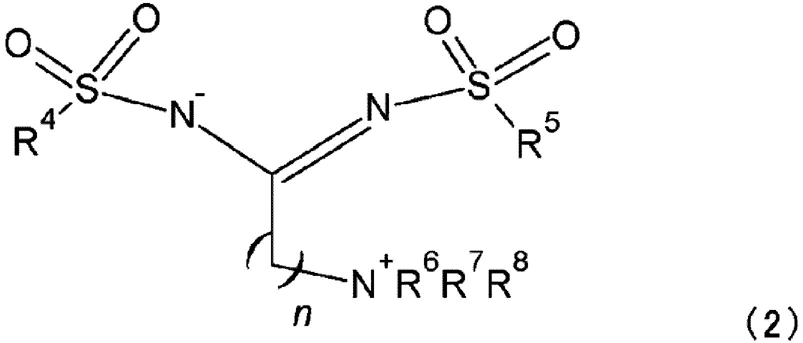
where R⁴ and R⁵ each independently represent a halogen atom, a perfluoroalkyl group having 1 to 4 carbon atoms, or a hydrocarbon group having 1 to 10 carbon atoms, where the hydrocarbon group may have a hetero atom or a halogen atom and may have a branched chain or a cyclic structure if the number of carbon atoms is 3 or more,
R⁶ to R⁸ each independently represent a hydrogen atom or a hydrocarbon group having 1 to 10 carbon atoms, where the hydrocarbon group may have a hetero atom or a halogen atom and may have a branched chain or cyclic structure if the number of carbon atoms is 3 or more,
n represents an integer of 1 to 4, and 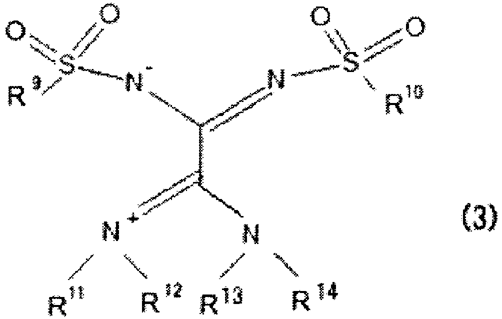
where, R⁹ and R¹⁰ each independently represent a halogen atom, a perfluoroalkyl group having 1 to 4 carbon atoms, or a hydrocarbon group having 1 to 10 carbon atoms, where the hydrocarbon group may have a hetero atom or a halogen atom, and may have a branched chain or a cyclic structure if the number of carbon atoms is 3 or more,
R¹¹ to R¹⁴ each independently represent a hydrogen atom or a hydrocarbon group having 1 to 10 carbon atoms, where the hydrocarbon group may have a hetero atom or a halogen atom and may have a branched chain or cyclic structure if the number of carbon atoms is 3 or more, R¹² and R¹³ may bond to each other to form a ring.

17. The compound according to claim 16,
wherein at least one of R¹ and R² in the Formula (1) represents a fluorine atom.

18. The compound according to claim 16,
wherein at least one of R⁴ and R⁵ in the Formula (2) represents a fluorine atom.

19. The compound according to claim 16,
wherein at least one of R⁹ and R¹⁰ in the Formula (3) represents a fluorine atom.
